# EUROPÄISCHE PATENTSCHRIFT

(11) **EP 1 575 654 B1**
(45) Veröffentlichungstag und Bekanntmachung des Hinweises auf die Patenterteilung: **18.03.2009**
(21) Anmeldenummer: 03767779.6
(22) Anmeldetag: 10.12.2003
(51) Int. Cl.: A61M 27/00

(54) **EINSTELLBARES HYDROCEPHALUSVENTIL**
ADJUSTABLE HYDROCEPHALUS VALVE
VALVE D'HYDROCEPHALIE REGLABLE

(30) Priorität: 11.12.2002 DE 10258070; 08.10.2003 DE 10347278
(43) Veröffentlichungstag der Anmeldung: 21.09.2005
(73) Patentinhaber: Christoph Miethke GmbH & Co. KG, 14469 Potsdam (DE)
(72) Erfinder: MIETHKE, Christoph, 14532 Kleinmachow (DE)
(74) Vertreter: Kaewert, Klaus
(86) Internationale Anmeldenummer: PCT/EP2003/013999
(87) Internationale Veröffentlichungsnummer: WO 2004/052443

(56) Entgegenhaltungen:
- EP-A- 0 421 557
- EP-A- 1 380 317
- US-A- 4 676 772
- US-A- 5 637 083
- US-A- 5 643 194

## Beschreibung

Die Erfindung betrifft ein perkutan verstellbares Hydrocephalusventil zum Druckausgleich im Schädel eines Hydrocephaluspatienten durch Drainage von überschüssigem Hirnwasser ins Peritoneum (Bauchhöhle) oder in das Herz des Patienten.

Hydrocephaluspatienten haben folgendes medizinisches Problem:

Das Gehirn ist von einer speziellen Flüssigkeit - dem Liquor - umgeben. Dieses Hirnwasser wird in symmetrisch angeordneten Hirnkammern gebildet, strömt durch Kanäle in den äußeren Liquorraum und wird dort wieder resorbiert. Normalerweise besteht ein Gleichgewicht zwischen der produzierten und der resorbierten Flüssigkeitsmenge. Bei der Erkrankung des Hydrocephalus (auch Wasserkopf genannt) ist eben dieses Gleichgewicht gestört, es wird nicht mehr ausreichend Flüssigkeit abgebaut. Das führt zu einem Druckanstieg im Kopf des Patienten. Beim Säugling führt dies dazu, dass der Kopf abnorm wachst, das Gehirn wird abgebaut und das Schädelinnere des Patienten ist zunehmend mit nur noch Hirnwasser gefüllt. Beim Erwachsenen ist ein Wachstum des Kopfes nicht mehr möglich, hier kommt es sehr schnell zu kritischen Druckwerten. Das Gehirn wird abgebaut (verwässert). Beim Erwachsenen kann dies zu Schwierigkeiten beim Gehen, Harninkontinenz und Demenz führen.

Zur Behandlung des Hydrocephalus hat sich seit den 50er Jahren die Implantation eines künstlichen Drainagesystems bewährt. Hierbei wird eine künstliche Verbindung von den Kammern im Gehirn zu einem Ableitungsmedium geschaffen. Heute wird in der Regel das Peritoneum (die Bauchhöhle) gewählt. Alternativ ist die Ableitung in den Vorhof des rechten Herzens auch üblich. In diese Systeme wurden spezielle Ventile integriert, die die Flüssigkeitsabfuhr kontrollieren sollen. Seit der Einführung der künstlichen Drainage zur Hydrocephalustherapie Anfang der 50er Jahre sind zahlreiche unterschiedliche Ventilsysteme vorgeschlagen worden, um das Behandlungsergebnis zu optimieren.

In den letzten Jahren sind zunehmend zwei konkurrierende Ventilprinzipien in das Augenmerk der Fachleute gerückt: einerseits perkutan verstellbare Ventile, bei denen die Öffnungscharakteristik an individuelle Patientenbedürfnisse angepasst werden kann, und andererseits Ventile, die eine sich lagebedingt ändernde Öffnungscharakteristik garantieren.

Letztgenannte Ventilsysteme haben in der Stehendposition des Patienten einen signifikant höheren Öffnungsdruck als in der Liegendposition. Beide Ventilsysteme werden heute sehr erfolgreich zur Therapierung des Hydrocephalus verwendet.

Wünschenswert ist die Kombination beider Ventiltypen. Im Rahmen der Erfindung wird eine solche Kombination nun konkret vorgeschlagen, wobei die bekannten Probleme bisher verfügbarer, verstellbarer Ventilsysteme gelöst werden.

Die wesentlichen Probleme der bekannten verstellbaren Ventile liegen in der Einstellgenauigkeit und in der Sicherheit gegen unintendiertes Verstellen des Ventilsystems durch von außen anliegende Magnetfelder. Alle bisher verfügbaren, verstellbaren Ventile basieren auf einem magnetischen Prinzip. Ventilseitig sind Magnete unterschiedlicher Größe an einem drehbar gelagerten Rotor angebracht. Durch von außen erzeugte Magnetfelder lassen diese Rotoren sich in ihrer Stellung beeinflussen, was zu einer Verstellung einer Federvorspannung führt.

Zu den bekanntesten Ventilen gehört das Medos-Codman-Ventil. Dieses Ventil enthält eine Blattfeder, die an der einen Seite auf einer Rubinkugel aufliegt, die durch die Blattfeder in den Ventilsitz und auf der anderen Seite auf einem treppenförmigen, drehbaren Rotor gedrückt wird. Zwischen diesen beiden Punkten - etwa in der Mitte - ist die Befestigung der Blattfeder angeordnet. Wird nun der Rotor durch Magnetfelder von außen gedreht, verändert sich der Auflagepunkt und die Öffnungscharakteristik des Ventils wird somit verstellt. Der Einstellbereich liegt zwischen 3 und etwa 20 cm Wassersäule. Der Außendurchmesser des Rotors beträgt etwa 3 mm. Der Verdrehbereich dieses Ventils beträgt 360 °. Dieses Ventil zeigt folgende systematische Problemfelder: Die Einstellung wird unintendiert durch außen anliegende Magnetfelder verändert. Eine Kontrolle dieser Veränderung kann nur mit Hilfe von Röntgenaufnahnnen erfolgen. Gerade für betroffene Patienten häufig notwendige MRT-Untersuchungen, insbesondere für Säuglinge, ziehen somit die röntgenologische Untersuchung der eingestellten Druckstufe nach sich. Dies ist belastend für den Patienten und limitiert somit den Einsatz dieses Ventiltyps.

Auch das verstellbare SU 8 von Sophysa - einer französischen Firma - zählt zu den bekannten verstellbaren Ventilen. Auch hier sind ventilseitig Magnete an einem drehbaren Rotor angeordnet. Die Position des Rotors kann durch Magnetfelder von außen verändert werden.

Saphirkugel drückt und dadurch die Ventilcharakteristik definiert. Wird der Rotor gedreht, verändert sich die resultierende Einspannlänge der Blattfeder und somit der Öffnungsdruck. Eine kurze Einspannlänge hat einen hohen Öffnungsdruck zur Folge, eine lange Einspannlänge einen niedrigen Öffnungsdruck. Der Verstellbereich liegt auch hier zwischen 3 bis 20 cm Wassersäule. Der verstellbare Drehbereich liegt bei etwa 90°, d. h., dass die Einstellung von 0 ° beispielsweise den minimalen Druck sicherstellt, die Einstellung von 90 ° den maximalen Druck. Die wesentlichen Schwachstellen dieser Konstruktion bestehen einerseits eben in diesem geringen Verstellbereich, da die Genauigkeit eines solchen Systems umso geringer wird, je geringer der verwendete Drehwinkel ist. Auch dieses Ventil hat den Nachteil, dass Magnetfelder von außen eine ungewollte Verdrehung und Verstellung der Öffnungscharakteristik nach sich ziehen. Bei dieser Konstruktion ist allerdings eine Röntgenkontrolle nicht erforderlich. Dafür ist allerdings die ungewollte Verstellung extrem einfach zu gewährleisten, beispielsweise durch Magnetfelder von Kopfhörern oder einfache Tafelmagnete.

Auf Grund dieser bekannten Schwierigkeiten hat die Firma Sophysa kürzlich eine neue Entwicklung vorgestellt, das so genannte Polaris-Ventil. Dieses Ventil ähnelt dem bekannten Ventil weitgehend. Allerdings ist zusätzlich ein magnetisch aktivierbarer Verblockungsmechanismus vorgesehen, der dazu führt, dass nur eine ganz spezifische Anordnung von extern angebrachten Magneten eine Verdrehung des Rotors möglich macht.

Ein drittes verstellbares Ventil wird von der Firma Medtronic (PS Medical) am Markt angeboten. Es handelt sich um das so genannte Strata-Ventil. Ähnlich den genannten Prinzipien sind ventilseitig zwei Magnete angebracht, die in einen Rotor integriert sind. Magnetfelder von außen ermöglichen die Verdrehung dieses Rotors und die Verstellung der Öffnungscharakteristik des Ventils. Dieses Ventil lässt sich in vier Stufen verstellen. Die Verdrehung des Rotors erfolgt durch Heranziehen des Rotors an das außen anliegende Magnetfeld und anschließende Rotation. Erst wenn der Rotor angezogen wird, ist eine Verdrehung möglich. Auch dieses Ventil enthält also eine Art magnetische Rasterung. Durch die Verdrehung dieses Rotors wir die Einbauhöhe des gleichen verändert, je höher der Rotor auf einer Art Treppe gelagert ist, umso höher ist der Öffnungsdruck. Ein solcher Mechanismus schränkt die Möglichkeiten der unintendierten Verstellung vielleicht ein, kann aber niemals sicher funktionieren.
Allen dargestellten Ventilen sind folgende Probleme einzeln oder zu mehreren gemeinsam:
1. Einstellungsfehler
2.Lesefehler hinsichtlich des eingestellten Öffnungsdruckes
3.nicht intendierte Verstellung
4.Funktionsschwächen

Die Erfindung hält gleichwohl an der bekannten Bauweise fest und hat sich die Aufgabe gestellt, ein Ventil zu schaffen, daß handhabungsfreudnlich und sicher ist. Dieses Ventil soll nach wie vor eine innen liegende, perkutan zu betätigende Verstelleinrichtung besitzen, die mit einer auußen liegenden Verstelleinrichtung bewegt wird.

Zwar ist mit der US-A4676772 ein Hydrocephalusventil bekannt, das für sich eine Sicherheit gegen unbeabsichtigte Verstellung beansprucht. In der Praxis wird der Anspruch aber nicht erfüllt:
Diese Druckschrift wird als nächstkommender Stand der Technik angesehen und zeigt ein Ventil zum Druckausgleich des Liquor im Schädel eines Hydrocephaluspatienten, wobei das Ventil dem Patienten implantiert ist und über eine gleichfalls implantierte Schlauchleitung der überschüssige Liquor aus den Hirnkammern im Schädel des Patienten ableitbar ist und in die obere Hohlvene oder in den Bauchraum drainiert werden kann. Dabei soll der Ventildruck durch eine Feder bestimmt werden und die Feder über eine Verstellmechanik verstellbar sein, Die Verstellmechanik besitzt ein schwenkbewegliches oder drehbewegliches Teil, das perkutan mittels Magneten verschwenkt oder gedreht werden kann, so daß die Feder gespannt oder entlastet wird.
Als Arretierung des durch die Magneten verstellbaren Teiles sind weitere Magneten vorgesehen, die wesentlich schwächer als die Verstellmagneten sind, aber ausreichen sollen, um das verstellbare Teil reibungsschlüssig anzuziehen.
Dies kann aber nur gelingen, solange die auf das verstellbare Teil wirkenden Kräfte für eine Verstellung zu gering sind und die Magnetkräfte nicht durch äußere Einwirkungen ganz oder teilweise aufgehoben werden.
Insbesondere die äußere Einwirkung ist zu beachten. In Häusern in Fahrzeugen, beim Telefonieren, in einer Vielzahl von Lebenssituationen sind die Menschen mehr oder weniger starken Magnetkräften ausgesetzt, die teilweise sogar wesentlich stärker als die in der Druckschrift vorgesehenen schwachen Magnete sind.

Nach der Erfindung wird eine ausreichende Sicherung mit den Merkmalen des geltenden Hauptanspruches erreicht. Dabei wird eine Federkraft zur Sicherung der Ventilstellung genutzt, wobei das Ventilgehäuse als Feder genutzt wird.

Das Ventilgehäuse kann dazu eine federnde Gehäusewand und einen an der Gehäusewand mittig in bestimmtem Abstand gehaltene, drehbewegliche Verstellung besitzen. Die Verstellung kann als Verstellteller oder als einarmiger oder mehrarmiger Hebel, Rot oder Formteil ausgebildet sein. Im weiteren wird nur von Verstellteller gesprochen. Das schließt andere Ausbildungen ein.

Der Abstand zur Gehäusewand ist dann so gewählt, daß der Verstellteller im unbetätigten Zustand mit dem Außenrand gegen die Gehäusewand gezogen bzw. gedrückt wird. Die damit verbundene Spannung in der Gehäusewand entsteht, wenn die Gehäusewand bei der Montage des Verstelltellers eingedrückt wird. Nach der Montage kann die Gehäusewand sich aufgrund des Verstelltellers nur noch teilweise in die ursprüngliche Form zurückbilden. Aufgrund des Federmaterials der Gehäusewand ist die Gehäusewand gleichwohl bemüht, sich in die vor der Montage bestandene Ausgangsform zurückzubilden. Das verursacht den gewünschten Andruck des Verstelltellers an der Gehäusewand.

Die Gehäusewand kann gerade sein und durch die oben beschriebene
Verformung/Eindrückung eine konkave Form einnehmen.
Die Gehäusewand kann auch ursprünglich konkav gewölbt sein und durch die oben beschriebene Verformung/Eindrückung eine weitere Einwärtswölbung erfahren.
Die Gehäusewand kann auch ursprünglich konvex sein und durch die
Verformung/Eindrückung eine teilweise Verringerung der Auswölbung oder eine vollständige Verringerung der Auswölbung erfahren. Wahlweise entsteht auch aus einer konvexen Ausgangsfom durch die Verformung/Einwärtswölbung eine Einwärtswölbung erfahren.

Die Andruckkraft kann durch die Dicke der Gehäusewand, durch das Maß der ursprünglichen Ausbildung, durch das Material der Gehäusewand und durch den Abstand des Verstelltellers von der Gehäusewand beeinflußt werden. Eine weitgehende Optimierung des Andruckes kann mit wenigen Versuchen erreicht werden.

Wie unter b) erläutert, kann die Andruckkraft zusätzlich oder anstelle von Feder auch durch Permanentmagnete der im Ventil liegenden Verstelleinrichtung verursacht werden. Bei Verwendung eines auf die Magnetfelder der Permanentmagnete reagierenden Werkstoffes für die Gehäusewand entsteht ein Andruck des Verstelltellers, der in Abhängigkeit von der Leistungskraft der Permanentmagneten steht. Als Material für die Gehäusewand ist Stahl besonders geeignet.

Vorzugsweise wird die Arretierung des Ventils im unbetätigten Zustand noch beeinflußt
c) durch Wahl des Durchmessers des Verstelltellers und/oder
d) durch die Beschaffenheit der Reibungsflächen

Eine höhere Reibung läßt sich durch rauhe Reibungsflächen erreichen. Wahlweise sind die Arretierungsflächen dazu aufgerauht oder mit einem reibungsintensiven Material beschichtet. Je größer der Durchmesser ist, auf dem die Reibungsflächen an der Gehäusewand liegen, desto größer ist die Arretierungswirkung.
Günstig ist auch eine Bauform, in welcher der Verstellteller im Querschnitt eine mehr oder weniger ausgeprägte U-form einnimmt. Dann korrespondiert die Gehäusewand mit einer am Außenrand des Verstelltellers vorgesehenen ringförmigen Erhebung.

Vorzugsweise wird der erfindungsgemäße Reibungsschluß aufgehoben
e) durch einen Gegendruck

Mit dem Gegendruck kann zwischen die Reibungsflächen ein ausreichender Abstand gebracht werden, um die im Ventil liegende Verstelleinrichtung frei zu bewegen.

Der zur Aufhebung des Reibungsschlusses erforderliche Gegendruck wird vorzugsweise von Hand aufgebracht und kann entstehen durch
f) durch Verformung der für den Federdruck nach a) ursächlichen Feder und/oder
g) durch Aufhebung der Magnetkraft

Der Gegendruck wird bei einer die Feder bildenden Gehäusewand dadurch erzeugt, daß von außen gegen die Gehäusewand gedrückt wird. Der notwendige Gegendruck kann werksseitig bestimmt und bei einer Ventilverstellung kontrolliert werden.
Für die Kontrolle eignen sich
h) mechanische Meßeinrichtungen
i) elektrische Meßeinrichtungen

Eine rein mechanische Meßeinrichtung kann durch einen Verstellstift gebildet werden, der eine federnd gelagerte Spitze besitzt. Durch Kontrolle des Federweges kann der notwendige Gegendruck kontrolliert und zugleich ein übermäßiger Gegendruck verhindert werden.

Die elektrische Meßeinrichtung kann zum Beispiel einen stromdurchflossenen Dehnungsmeßstreifen nutzen. Der Dehnungsmeßstreifen verändert je nach Belastung seinen Widerstand, so daß sich der Stromfluß oder die Spannung verändert. Durch Messung der Veränderung wird der Gegendruck gemessen. Das kann zugleich genutzt werden, um bei Erreichen eines Mindest-Gegendruckes ein optisches und/oder akustisches Signal zu geben, das solange anhält wie der Maximal-Gegendruck nicht überschritten wird oder das bei Überschreiten des Maximal-Gegendruckes durch eine zusätzliches optisches und/oder akustisches Signal ergänzt wird.

Wahlweise wird der Gegendruck auf das zulässige Maß begrenzt durch:
j) ein zwischengeschaltetes Federglied

Das Federglied ist dann in der außen liegenden Verstelleinrichtung vorgesehen. Das Federglied besteht wahlweise aus einer langhubigen, vorgespannten Feder. Bei dieser Verstelleinrichtung wird bei entsprechender Auslegung der Federvorspannung eine Nachgiebigkeit der Verstelleinrichtung merklich, wenn der notwendige Druck erreicht ist, bei dem der Verstellteller von seinen zugehörigen Reibungsflächen abhebt. Der zulässige Druck wird erst überschritten, wenn die Nachgiebigkeit der außen liegenden Verstelleinrichtung nicht mehr spürbar ist. Dazwischen ist ein erheblicher Nachgiebigkeitsbereich vorgesehen, in dem sich der behandelnde Arzt bei der Handhabung des Ventiles sicher sein kann, in dem Bereich zulässigen Druckes zu sein.

Vorzugsweise erfolgt die Ventilverstellung nach Lösen des Verstelltellers:
k) Drehen des Verstelltellers

Noch weiter bevorzugt ist ein großer Verstellweg zwischen der minimalen Öffnungsstellung und der maximalen Öffnungsstellung des Ventils vorgesehen. Das erhöht die Verstellsicherheit und die Verstellgenauigkeit.
Der große Verstellweg wird in eine Änderung der Federbelastung untersetzt. Das heißt, bei vergleichbarem Änderungsbereich der Federbelastung ist ein größerer Verstellweg vorgesehen. In dem Umfang, in dem der Verstellweg größer wird, verringert sich die oben wiedergegebene Gefahr unerwünschter Verstellung.
Vorteilhafterweise erhöht sich zugleich die Genauigkeit der Verstellung mit größer werdendem Verstellweg.

Die Möglichkeit zur größeren Gestaltung des Verstellweges ergibt sich durch Änderung der Lage der Feder. Nach der Erfindung wird die Feder so gelegt, daß deren Bewegungsebene parallel zur Dreh-Ebene des Verstelltellers liegt. Im erfindungsgemäßen Sinne ist die Parallelität auch dann gegeben wenn die Ebenen zusammenfallen.
Durch die erfindungsgemäße Anordnung der Feder kann die Feder sich in der Richtung bewegen, in der sich das Ventilgehäuse am weitesten ausdehnt. Das ist die Richtung der Flachseite.

Wahlweise findet als Feder ein Federstab Anwendung, der schwenkbeweglich angeordnet ist. Der Federstab kann einen doppelarmigen Hebel bilden, dessen eines Ende länger als das andere Ende ist. Dadurch findet nach Wahl eine Übersetzung oder Untersetzung der Hebelbewegung bzw. der Hebelkraft statt. Das eine Hebelende steht mit einer Ventilkugel oder Ventilklappe des Ventils in Wirkverbindung, das andere Hebelende wirkt mit der beschriebenen Verstellmechanik bzw. dem Verstellteller zusammen.

Dabei ist vorzugsweise eine gleitende, an sich bekannte Wirkverbindung zwischen der Feder und der zugehörigen Fläche an dem Verstellteller vorgesehen. Das heißt, die Feder gleitet auf der Berührungs-Fläche des Verstelltellers.

Die Wirkverbindung mit der Ventilkugel bzw. der Ventilklappe wird dadurch gebildet, daß das kürze Ende gleitend gegen die Ventilkugel bzw. die Ventilklappe drückt.
Die Wirkverbindung mit der Verstellmechanik mit der Verstellmechnik wird dadurch gebildet, daß an dem drehbeweglichen oder schwenkbeweglichen Teil eine Gleitfläche für den anderen Hebelarm vorgesehen ist. Diese Gleitfläche ist als Kurvenbahn ausgebildet, an welcher der Federstab gleitend anliegt. Die Kurvenbahn erlaubt es, dem Ventil in weiten Grenzen eine einstellbare Ventilcharakteristik zu geben. Die Kurvenbahn verläuft vorzugsweise zumindest teilweise spiralförmig. Der Umfangswinkel der Kurvenbahn an der Gleit-Berührungsfläche des Verstelltellers umfaßt vorzugsweise mindestens 300 Grad, noch weiter bevorzugt mindestens 340 Grad.

Je nach den Hebelverhältnissen wird die Bewegung des an der Kurvenbahn des Verstelltellers anliegenden Hebelarmes auf den anderen, an der Ventilkugel oder Ventilklappe anliegenden Hebelarm übersetzt oder untersetzt.

Je nach Auslegung der Ventilkugel oder der Ventilklappe kommt es dabei auf ein Zurückweichen oder Anstellen des korrespondierenden Hebelarmes an. Oder es kommt auf eine Änderung der Andruckkraft des an der Ventilkugel oder Ventilklappe anliegenden Hebelarmes an.

Die Drehrichtung des Verstelltellers bestimmt die Schwenkrichtung des Hebels. Bei hin- und hergehender Bewegung des Verstelltellers ergibt sich auch eine hin- und hergehende Schwenkbewegung des Hebel in Richtung einer minimalen Öffnungweite oder eines minimalen Schließdruckes am Ventil oder umgekehrt in Richtung der maximalen Öffnungsweite oder des maximalen Schließdruckes.

Wahlweise kann der Verstellteller auch in der gleichen Drehrichtung weiterbewegt werden und gleichwohl wieder an den Verstellanfang kommen. Das wird dadurch erreicht, daß zwischen dem Anfang der Kurvenbahn und dem Ende der Kurvenbahn an dem drehbeweglichen oder schwenkbeweglichen Verstellteller ein Übergang vorgesehen ist.

Aus obigem ergibt sich, daß das erfindungsgemäße Ventil sowohl zur Erlangung einer Ventilstellung auf dem kürzesten Weg in die neue Ventilstellung als auch in entgegen gesetzter Drehrichtung bis zum Ende der Kurvenbahn und darüber hinaus in die neue Ventilstellung gedreht werden kann. Letzteres kann gewünscht sein, wenn jede Ventileinstellung zur Kontrolle von einem Verstellanfang bzw. Anfang der Kurvenbahn ausgehen soll. Der doppelarmige erfindungsgemäße Feder-Hebel hat vorzugsweise eine Winkelform. Die beiden Hebelarme des doppelarmigen Hebelarmes stehen in einem Winkel zueinander, der kleiner als 180 Grad ist, auch kleiner als 90 Grad sein kann.

Der Querschnitt der erfindungsgemäßen Feder kann beliebig sein. Günstig sind runde und rechteckige Formen. Besonders günstig ist eine Feder mit blattförmigem oder drahtförmigem Querschnitt.

Zur schwenkbeweglichen bzw. drehbeweglichen Lagerung der Feder eignet sich zum Beispiel ein Stift, dessen Enden in entsprechende Ausnehmungen im Ventilgehäuse bzw. im Ventildeckel oder Ventilboden greifen. Die Enden des Stiftes können auch spitz ausgebildet sein, so daß der Stift in den Ausnehmungen auf den Spitzen dreht. Diese Vorgehensweise ist technisch und wirtschaftlich günstig.

Zur Befestigung des Stiftes an der Feder eignet sich eine Schweiß- oder Lötverbindung. Es eignen sich auch andere Verbindungen.

Die erfindungsgemäße Feder kann auch bei Verwendung eines Federdrahtes ohne weiteres auf der Kurvenbahn des Verstelltellers aufliegen.

Ventilkugelseitig ist es günstig, wenn eine großflächige Berührung zwischen der Feder und der Ventilkugel stattfindet. Soweit die Feder diese großflächige Berührung nicht hergibt, kann an dem betreffenden Federende ein Blech befestigt werden. Das Blech ist wahlweise angeschweißt oder angelötet oder in sonstiger Weise befestigt.

Die für die Verstellung des Ventiles erforderliche Drehung des Verstelltellers findet vorzugsweise mittels zusätzlichen außen angeordneten Magneten und einer außen liegenden Dreheinrichtung statt. Die Magneten sind vorzugsweise Permanentmagneten. Noch weiter bevorzugt sind mindestens zwei Magneten in dem Verstellteller diametral gegenüberliegend und mindestens gleich viele Magneten in der Dreheinrichtung gleichermaßen gegenüberliegend angeordnet, wobei die Magneten des Verstelltellers auf einer Kreisbahn liegen, deren Durchmesser gleich dem Durchmesser der Kreisbahn ist, auf dem die Magneten der Dreheinrichtung liegen. Dadurch können die verschiedenen Magneten einander möglichst nahe gebracht werden. Durch die räumliche Nähe können die Magnete optimale Kräfte entfalten. Die für die Entstehung eines Drehmomentes erforderlichen Drehmomente entstehen, wenn die Magnete einander mit unterschiedlichen Polen gegenüberliegen, z.B. mit einem Südpol in der Dreheinrichtung und mit einem Nordpol in dem Verstellteller.

Vorteilhafterweise können bei der erfindungsgemäßen Vorrichtung auch relativ kleine Magnete ein ausreichendes Drehmoment für die Ventilverstellung entfalten.

Zweckmäßig wird die Dreheinrichtung zugleich als Verstelleinrichtung genutzt. Dazu findet eine unten beschriebene Vorrichtung Verwendung, mit der nicht nur die oben beschriebene Drehung sondern auch der oben beschriebene Druck ausgeübt werden kann.

Nach einer Verstellung des Ventiles durch Drehung des Verstelltellers findet eine Arretierung des Verstelltellers in der jeweiligen Drehstellung statt. Die Arretierung entsteht, wenn die zuvor eingedrückte Gehäusewand wieder entlastet wird. Dann entstehen erneut der oben beschriebene Andruck und die damit verbundene Reibung.

In der Arretierungsstellung des Verstelltellers verstärken die Magneten die Arretierung, indem sie den Andruck und die Reibung zwischen dem Verstellteller und dem Ventilgehäuse erhöhen. Dazu ist vorzugsweise die den Magneten nächste Fläche als eine reaktive Metallfläche ausgebildet. Besonders reaktiv sind Stahlflächen.

Die erfindungsgemäß mit den im Ventil eingeschlossenen Magneten bewirkten zusätzlichen Arretierungskräfte stören vorteilhafterweise die Ventilverstellung mit außen liegenden Magneten nicht, weil durch den oben beschriebenen Gegendruck die Magnetkraft leicht überwunden und der notwendige Abstand des Verstelltellers von der korrespondierenden Gehäusewand hergestellt werden kann.

Als Magnete finden vorzugsweise kleine Bauformen als Stiftmagnete gemäß den Patentansprüchen Verwendung. Die kleinen Magnete tragen auch zu geringen Ventilabmessungen bei, wie sie Gegenstand der Patentansprüche sind.

Die Verstelleinrichtung für das erfindungsgemäße Ventil kann gleichfalls mit extrem kleinen Abmessungen gestaltet werden. Nach der Erfindung wird das zur Reduzierung des Durchmessers der Verstelleinrichtung und zu einer besonderen Formgebung der Verstelleinrichtung genutzt, nämlich zur Gestaltung der Verstelleinrichtung in Stiftform, ähnlich einem Kugelschreiber. Die einem Kugelschreiber ähnliche Ausbildung der Verstelleinrichtung erlaubt die Handhabung der Verstelleinrichtung wie die Handhabung eines Stiftes oder Kugelschreibers, z. B durch Tragen in einer Brusttasche. Zugleich kann eine Mechanik wie bei einem Kugelschreiber genutzt werden, um die am Kopf der Verstelleinrichtung vorgesehenen Magnete in Längsrichtung des Stiftes vor (bei aufgesetztem Stift gegen den Kopf des Patienten bzw. gegen das Ventil) oder zurück zu bewegen. Bei vertikaler Lage des Stiftes ist das ein Heben und Senken.

Wahlweise besitzt die erfindungsgemäße stiftförmige außen liegende Verstelleinrichtung am vorderen Ende eine Kappe, mit dem die Verstelleinrichtung aufgesetzt wird. Bei lockerem Aufsetzen der Verstelleinrichtung bewirken die Magneten selbsttätig eine Zentrierung der Verstelleinrichtung, so daß es leicht ist, die Verstelleinrichtung durch Drehen zu betätigen.

Die Vorteile des erfindungsgemäßen Ventils und seiner Verstelleinrichtung sind:
1. präzise Einstellung der Druckcharakteristik durch Verwendung eines möglichst großen Verstellwinkels
2. Auslesen des eingestellten Öffnungsdruckes ohne Röntgenerkennung
3. Verhinderung einer nicht intendierten Verstellung des Ventils
4. Erhöhung der allgemeinen Funktionssicherheit

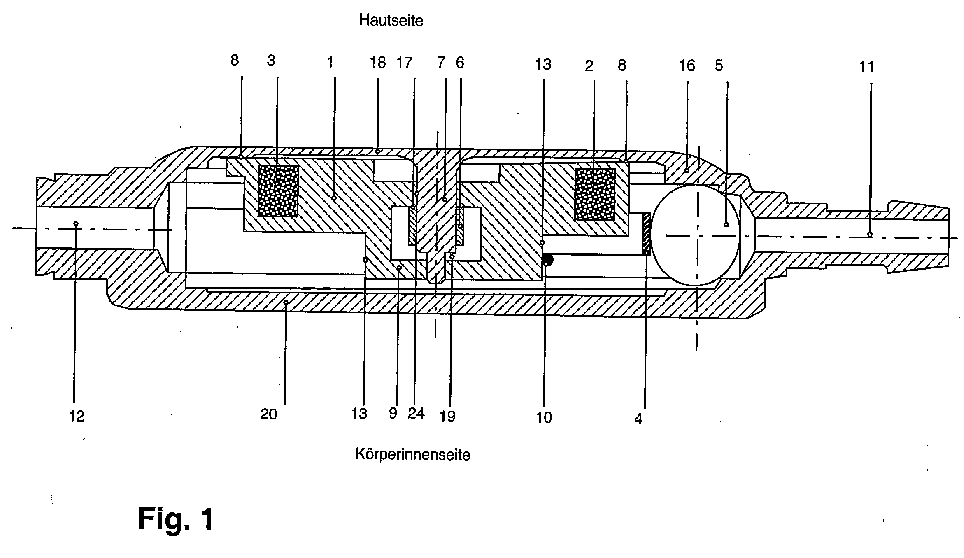

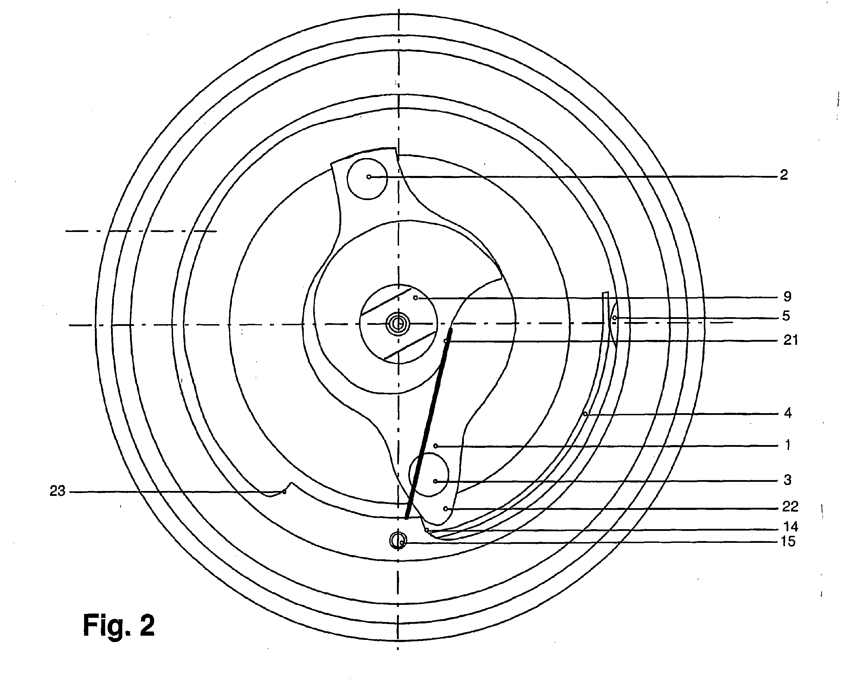

Figur 1 zeigt eine schematische Querschnittszeichnung der Erfindung in vielfacher Vergrößerung. Das Ventil besteht aus einem massiven Titangehäuse 16, in das eine Ventiltülle 11 eingebracht ist. Die Saphirkugel 5 wird durch die Blattfeder 4 in den Ventilsitz gedrückt. Die Blattfeder 4 bildet mit dem Federdraht 3 und einer in Figur 2 sichtbaren Achse 15 eine funktionale Einheit. In den als Rotor 1 bezeichneten Verstellteller sind zwei Magnete 2 und 3 mit umgekehrter Polung eingebracht. Der Rotor 1 ist auf einer Achse 7 gehalten. Die Achse 7 befindet sich an einem Boden 18 des Titangehäuses 16. Der Boden 18 ist gewölbt. Der Rotor 1 ist mit einer Schraube 6 auf der Achse 7 so gegen den Deckel gespannt, daß ein Anpressung des Rotors 1 am Boden 18 und dessen elastische Verbiegung erfolgt. Die Anpressung erfolgt so, dass die Reibkraft ausreicht, eine Verdrehung des Rotors 1 auf Grund von äußeren Magnetfeldern zu verhindern. Der Boden 18 hat vorzugsweise eine Stärke von 0,1 bis 0,2 mm, in anderen Ausführungsbeispielen eine Stärke bis 0,5mm. Die elastische Verbiegung verbundene Verformung beträgt vorzugsweise 0,01 mm bis etwa 0,1 mm, in anderen Ausführungsbeispielen bis zum zweifachen der Bodenstärke. Je stärker der Boden vorgespannt ist, desto stärker muss später von außen gedrückt werden, um ein Abheben des Rotors 1 bei 6 von dem Boden 18 zu bewirken und die Arretierung des Rotors 1 am Boden 18 aufzuheben.

Die Stellung des Rotors 1 definiert die Kraft, welche die Blattfeder 4 auf die Saphirkugel 5 ausübt.

In Figur 2 ist ein von unten geöffnetes Ventil dargestellt. Zu sehen ist hier die Feder 10, die verschweißt ist mit der Achse 15 und der Blattfeder 4. Diese Komponenten sind vorzugsweise aus einem metallischen Material hergestellt, insbesondere aus Titan oder einer Titanlegierung. Der Federdraht der Feder 10 hat vorzugsweise den Durchmesser 0,1 mm, in anderen Ausführungsbeispielen kann der Federdraht bei kürzeren Längen einen geringeren und bei größeren Längen einen größeren Querschnitt haben. Der Querschnitt des Federdrahtes ist im Ausführungsbeispiel kreisförmig. Die Blattfeder hat vorzugsweise eine Stärke von ebenfalls 0,1 mm und eine Höhe von etwa 1 mm. Für andere Ausführungsbeispiele mit kleineren Längen und mit größeren Längen gelten die Hinweise zum Draht der Feder 10 entsprechend. Die Blattfeder ist sehr steif.

Die Achse 7 besitzt in der Zeichnung links einen Absatz und Zapfen, mit dem sie in eine kleinere Bohrung des Teiles 9 am Rotor 1 ragt. Im Einbauzustand besteht zwischen der Achse 7 und dem Teil 9 ein Spalt an der Stelle 19.

In Figur 1 ist die Hautseite in der Zeichnung rechts und die Körperinnenseite in der Zeichnung links dargestellt. Wird nun von außen durch die Haut mechanisch ein Druck auf den Boden 18 ausgeübt, so wird dadurch in Abhängigkeit von der Kraft der Boden 18 nach - innen verformt/gewölbt und die Achse 7 nach unten zum Deckel 20 gedrückt. Hierdurch wird der Spalt 19 geschlossen, die Achse 7 drückt gegen Teil 9 und hebt somit den gesamten Rotor ab von dem Boden 18 ab. Die elastische Vorspannung des Bodens 18 wird und die Reibkräfte an der Stelle 8 werden dabei aufgehoben. Jetzt entsteht an der Stelle 8 ein Spalt, der Rotor ist nun frei drehbar. Wird die äußere Last wieder weggenommen, geht der äußere Boden 18 wieder in seine Ausgangslage zurück und erzeugt die elastische Vorspannung zwischen Auflagepunkt 17 und Auflagepunkt 8. Der Rotor wird wieder im Gehäuse geklemmt, eine Verdrehung ist nicht möglich.

Der Rotor 1 besitzt eine Kurvenscheibe 13.
In Figur 2 ist der Rotor 1 in der Minimalposition dargestellt. Durch Verdrehung um etwa 300 Grad wird die Feder 10 am Auflagepunkt 21 entsprechend der Kurvenscheibe 13 in ihre Maximalposition gebracht, so dass der resultierende Öffnungsdruck nun maximal wird. Der Höhenunterschied zwischen der minimalen und maximalen Federvorbiegung von Teil 4 oder 10 beträgt in etwa 0,7 bis 0,8 mm. Konkret richtet sich dies jedoch nach der Abmessung des gewählten Titandrahtes 10.

Die Anordnung der beiden Magnete 2 und 3 erfolgt so, dass ein außen anliegendes Magnetfeld ein maximales Drehmoment erzeugen kann.
D: h., der Abstand der beiden Magnete beträgt im Ausführungsbeispiel 7 mm, in einem anderen Ausführungsbeispiel 8 mm und in noch weiteren Ausführungsbeispielen bis 20mm. Konkret richtet sich dieser Abstand nach den Außenabmessungen des Gehäuses. Das kreisrunde Gehäuse hat vorzugsweise einen Durchmesser von 14mm, in anderen Ausführungsbeispielen bis 19 mm und in noch weiteren Ausführungsbeispielen bis 31mm und ist ergonomisch geformt, so das einerseits die Lage des Ventils von außen gut getastet werden kann, aber andererseits das über dem Ventil liegende Gewebe nicht geschädigt wird. Scharfe Kanten werden daher vermieden.

Der Rotor hat eine Spitze 22, in Figur 2 dargestellt. Diese Spitze schlägt gegen Anschlag 14 beim minimalen Wert und gegen Anschlag 23 beim maximalen Wert. Durch diesen Anschlag wird im Ausführungsbeispiel verhindert, dass Maximal- und Minimaleinstellungen unmittelbar ineinander übergehen und jederzeit gut unterscheidbar bleiben. In anderen Ausführungsbeispielen ist wahlweise ein Übergang vorgesehen.

Die Achse 15 hat vorzugsweise einen Durchmesser von 0,3 mm und kann ggf. oben und unten eine Spitze aufweisen, um die Lagerkräfte zu minimieren. Auf Grund der beschriebenen Bauweise ist eine Verdrehung des Rotors 1 nur möglich, wenn der Boden 18 in der Zeichnung nach links gepresst und der Rotor 1 dadurch frei drehbar wird. Gleichzeitig muss in diesem Zustand ein spezifisches Magnetfeld von außen angeordnet werden, um eine Verdrehung sicher zu stellen. Wird der Boden dann entlastet, wird die Position des Rotors durch elastische Klemmung fixiert. Entsteht nun zwischen dem Einlass 11 und dem Auslass 12 des Ventils ein Differenzdruck, der größer ist als der Öffnungsdruck des Ventils, wird die Kugel 5 aus ihrem Ventilsitz gegen die Blattfeder gedrückt und in Richtung des Rotors bewegt. Dadurch wird es möglich, dass Hirnwasser vom Einlass zum Auslass das Ventil durchströmt und ein weiterer Druckanstieg verhindert wird. Die tatsächliche Ventilcharakteristik wird definiert durch die Verdrehposition des Rotors 1 bzw. die daraus resultierende Position des Auflagepunktes 21 auf der Spirale bzw. der Kurvenscheibe 13. Durch gezielte Änderung der Form der Kurve kann in anderen Ausführungsbeispielen auch ein nicht linearer Verlauf der Öffnungscharakteristik in Abhängigkeit vom Verdrehwinkel des Rotors 1 eingestellt werden. Vorzugsweise ist der Rotor so gefertigt, dass in allen Ausgangspositionen des Rotors eine Verdrehung von 10 Grad in die eine oder andere Richtung die jeweils gleiche Änderung des Öffnungsdruckes des Ventils nach sich zieht. Die Anordnung der Magnete 2 und 3 möglichst weit auseinander hat den Vorteil, dass mit möglichst geringen Magnetkräften möglichst große Verstellmomente realisiert werden können. Die hier verwendeten Neodym-Magnete haben eine zylindrische Form mit einem Durchmesser von 1 mm und einer Höhe von etwa 1,2 mm. Die Fertigung von Gehäuse und Rotor sowie die Fertigung der anderen Komponenten aus Titan hat den Vorteil, mit präzisen Passungen ein ideales Lagerspiel einstellen zu können und ungewolltes Spiel ebenso wie ungewollt erhöhte Reibung systematisch zu vermeiden. So hat die Achse 7 vorzugsweise einen Durchmesser von 1 mm, das Spiel an Position 24 zwischen Achse 7 und Rotor 1 ist vorzugsweise durch eine enge Spielpassung toleriert. Eine eben solche Spielpassung ist vorgesehen zur Lagerung der Achse 15 im Ventilgehäuse. Diese Achse 15 ist ähnlich einer Türangel im Ventilgehäuse gelagert und ermöglicht die nahezu reibungsfreie Verdrehung der Blattfeder 4 im Rahmen des Öffnens und Schließens des Ventils. Die Bauhöhe des Ventils liegt bei etwa 4,5 mm, wesentlich niedrigere Bauhöhen sind nicht unbedingt wünschenswert, wenngleich möglich, da das palpatorische Auffinden des Ventils nicht zu schwierig sein soll.

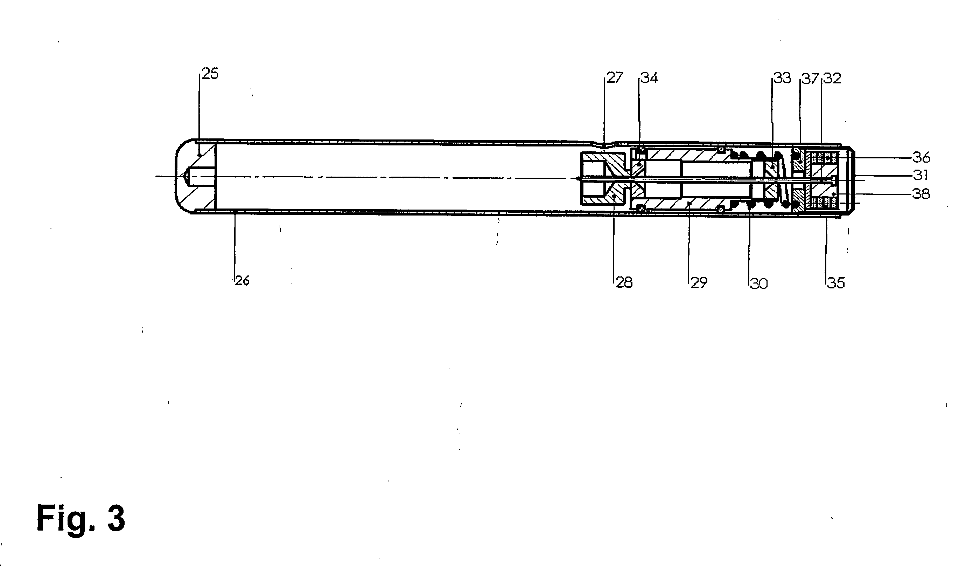

Für die Verstellung des Ventils sind spezielle Verstellstifte entwickelt worden. Ein Ausführungsbeispiel eines solchen Stiftes ist in Figur 3 dargestellt. Die Darstellung beinhaltet auch eine Vergrößerung gegenüber dem Ausführungsbeispiel, jedoch weniger groß als in Fig. 1 und 2. Um auf die richtigen Maßrelationen des Ventils zum Stift als Verstelleinrichtung zu kommen, wird empfohlen, den Stift in entsprechender Vergrößerung zusammen mit dem Ventil zu betrachten.
In maßstabsgetreuer Darstellung sind alle Einzelheiten so klein, daß sie nicht mehr erkennbar werden.

Ein dünnwandiges Röhrchen 26 mit einem Durchmesser von etwa 12 mm ist an einem Ende durch einen Stopfen 25 verschlossen. An der anderen Seite ist eine nadelgelagerte Messmechanik eingebaut. Dazu gehören: Messtrommel 28, auf deren Oberfläche eine Skalierung aufgebracht ist, ist mit der Achse 32 verbunden, die in der Lagerbuchse 29 an den Stellen 34 und 33 gelagert ist. Die Lagerbuchse 29 ist so in das Röhrchen 26 eingebracht, dass eine Verschiebung und Verdrehung derselben nicht möglich ist. An der nicht verschlossenen Seite des Röhrchens ist eine bewegliche Kappe in das Röhrchen eingeführt, die durch eine Federkraft nach außen gedrückt wird. Die Feder 30 stützt sich an der Lagerbuchse 29 ab und drückt den Ring 37 gegen die Kappe 31. Mit der Nadel 32 verbunden ist der Zylinder 38. In den Zylinder 38 eingebracht sind Magnete 35 und 36. Bei dem einen Magnet ist der außen liegende Pol negativ, bei dem anderen positiv. Der Abstand der Magnete entspricht in etwa dem Abstand der Magnete innerhalb des Ventils, ebenso der Durchmesser.

Durch die Kappe 31 und die Feder 30 ist ein Verdrehen der Achse des Rotors und des Zylinders 38 nicht möglich, solange nicht die Kappe gegen die Federkraft auf die Lagerbuchse gedrückt wird. Erst wenn der Stift oberhalb des Ventils gegen den Kopf des Patienten gedrückt wird und somit die Kappe 31 in das Stiftgehäuse drückt, ist eine Drehung von Rotor und Skalentrommel sowie Magnetzylinder möglich. Der Stift ist so gegen den Kopf des Patienten zu drücken, dass das Fenster 27 um 90 Grad verdreht zur Körperachse eingesehen werden kann. Hierdurch wird sichergestellt, dass Ventilstift und das Ventil selbst die gleichsinnige Orientierung haben. Wird nun die Kappe oberhalb des Ventils vor den Kopf des Patienten gedrückt, folgt die Stellung des Rotors innerhalb des Stiftes der Stellung des Rotors innerhalb des Ventils, da der Ventilrotor durch die elastische Klemmung in seiner Position nicht verändert werden kann, der Stiftrotor jetzt aber wegen der feinen
Nadellagerung an den Stellen 33 und 34 durch Verdrehung sich der Position des ventilseitigen Rotors anpassen kann. An dem Fenster 27 kann der entsprechende Einstellungsdruck des Ventils nun leicht ausgelesen werden. Diese Konstruktion gewährleistet ein sicheres, jederzeit leicht wiederholbares Messen. Durch die Fixierung des Messwertes nur wenige Zehntelmillimeter entfernt vom Kopf ist eine Verdrehung nach Wegnehmen des Stiftes vom Kopf des Patienten nicht mehr möglich. Das Messergebnis wird unmittelbar eingefroren.

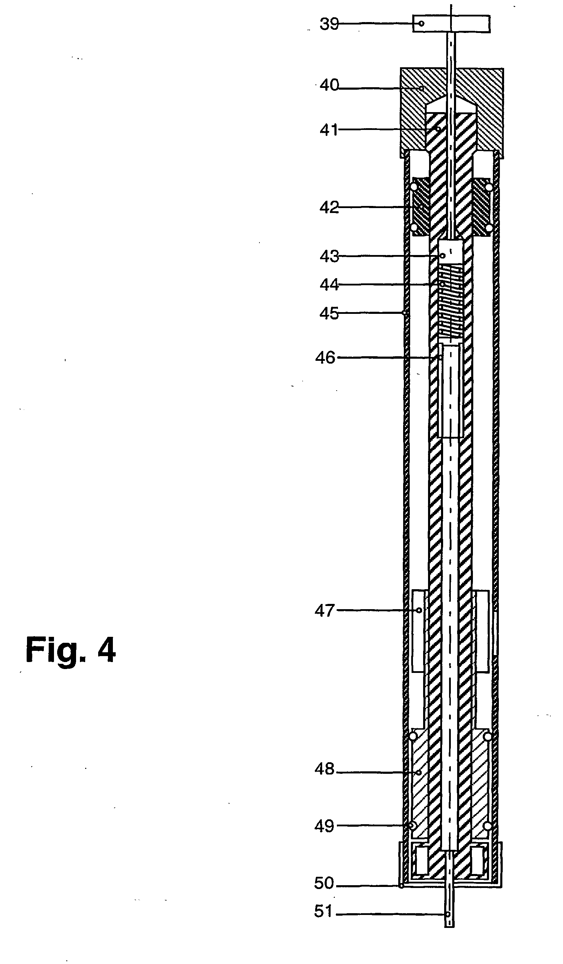

Figur 4 zeigt ein anderes Ausführungsbeispiel eines Verstellstiftes. Die Abmaße entsprechen in etwa den Maßen eines gewöhnlichen Kugelschreibers, d. h., das Röhrchen hat einen Außendurchmesser von vorzugsweise 12 mm und eine Länge von etwa 10 cm. Das Einstellrad 40 ist fest auf der Achse 41 fixiert. Eine Verdrehung dieses Rades bewirkt eine Verdrehung der Achse. Am unteren Ende der Achse 40 sind in die Achse zwei Magnete 50 zylinderförmig eingebracht. Wie im Ventil sind diese Magnete unterschiedlich gepolt. Bei dem einen Magneten liegt der Südpol unten, bei dem anderen liegt der Nordpol unten. Die Position der beiden Magnete auf der Achse korrespondiert mit der Position der Skalierung, die auf Teil 47 angebracht ist. Diese Skalierung ist ebenfalls fest mit der Achse verbunden. Die Buchse 48 dient als Lagerung für die Achse 41. Die Buchse wird eingebracht durch O-Ringe, durch die die Buchse in der Hülse 45 fixiert wird. Eine zweite Lagerbuchse ist am oberen Bereich des Stiftes angebracht, Teil 42. Auch hier ist Achse 43 als Gleitlagerung in Buchse 42 fixiert. Der Verstellstift enthält zwei verschiedene Federn: eine starke Feder 44 und eine extrem weiche Feder 46. Durch Druck auf Knopf 39 wird die Achse 43, die im unteren Bereich eine kolbenartige Erweiterung hat, gegen die Federkraft 44 nach unten verschoben. Hierdurch wird die Achse 51 gegen die Federkraft der deutlich weicheren Feder 46 nach unten verschoben. Die Feder 46 wird also stark komprimiert, wo hingegen die Feder 44 nur wenig komprimiert wird. Die Kraft der Feder 44 wird durch die Achse 51 auf deren untere Spitze übertragen, die im Anwendungsfall die Kraft auf das zu entkuppelnde Ventil ausüben soll. Der Durchmesser der Achse an der Spitze sollte vorzugsweise etwa 3 mm betragen, das untere Ende sollte kuppelförmig abgerundet sein. Die im unteren Ende des Stiftes angebrachte Kappe 51 schützt die Lagerung sowie die in der Achse 41 eingebrachten Magnete 50. Durch das Fenster 53 ist die Stellung der Magnete über die Skala der Skalentrommel 47 ablesbar. Durch die vorgeschlagene Konstruktion ist es möglich, die Verstelleinheit denkbar klein zu bauen, ohne die Verstellsicherheit negativ zu beeinträchtigen. Erstmals wird es möglich, solche Verstellstifte zu realisieren. Die Konstruktion ermöglicht ein möglichst nahes Platzieren der Magnete auf der Haut des Patienten. Unter gleichzeitiger Druckbelastung des Ventilgehäuses kann eine feine und präzise Verstellung vorgenommen werden.

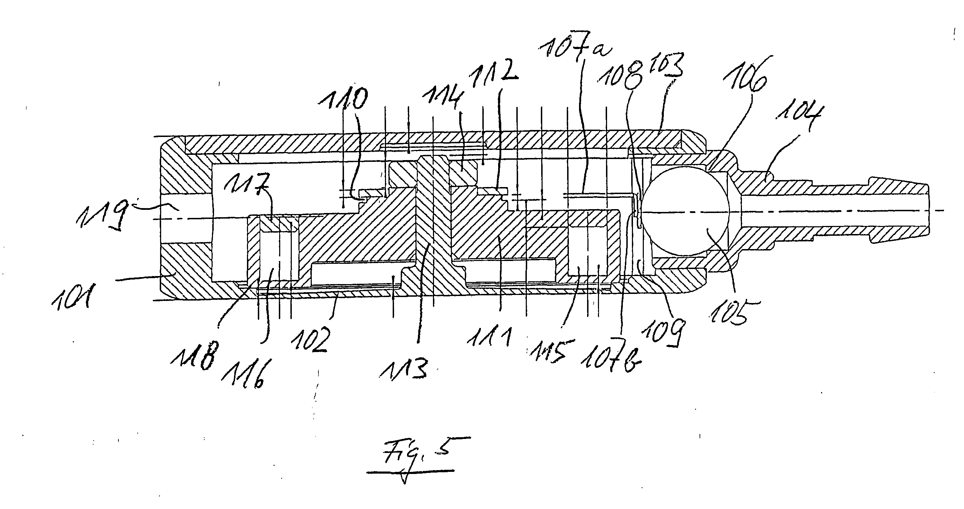

Die Fig. 5 bis 7 zeigen weitere Ausführungsbeispiele
Auch Fig. 5 zeigt einen Schnitt durch ein erfindungsgemäßes Ventil in vergrößerter Darstellung. Tatsächlich ist im Ausführungsbeispiel ein Durchmesser von weniger als 20 mm und eine Dicke von weniger als 6 mm vorgesehen. Die Abmessungen können in anderen Ausführungsbeispielen noch geringer sein.

Zu dem Ventil gehört ein Stahlgehäuse, das aus einem zylindrischen Ring 101, einem angeformten Stahlboden 102 und einem Deckel 103 besteht. Der Ring 101 ist zuflußseitig mit einem Einsatz 104 versehen. In dem Einsatz findet sich eine Ventilkugel 105, die in einer Ventilbohrung 106 dichtet. Die Ventilkugel 105 wird mit einer drahtförmigen Feder gegen die Ventilbohrung 106 gedrückt. Die Feder ist als doppelarmiger Hebelarm mit einem langen Hebelarm 107a und einem kleinen/kurzen Hebelarm 107b ausgebildet.
Die beiden Hebelarme 107a und 107b stehen in einem spitzen Winkel zueinander, weil der Schnitt durch die Ventilmitte verläuft und weil die Feder in dem Bereich dargestellt ist, der im Bild hinter der Schnittebene liegt und weil das freie Ende des Hebelarmes 107a in den nicht dargestellten Bereich verläuft, der vor der Schnittebene liegt und weil die Darstellung des kurzen Hebelarmes 107b auf einen Verlauf genau senkrecht zur Darstellungsebene und Schnittebene hinweist.
In anderen Ausführungsbeispielen sind andere Winkel vorgesehen.

Zwischen beiden Hebelarmen 107a und 107b ist die Feder an einem Stift 109 angelötet. Der Stift 109 ist seitlich von der Ventilkugel 105 angeordnet und besitzt zwei spitze Enden, mit denen er im zylindrischen Ring 101 schwenkbeweglich gelagert ist.
An dem kurzen Hebelarm 107b ist ein Blech 108 befestigt, mit dem die Feder gegen die Ventilkugel 105 drückt. Je mehr die Feder drückt, desto größer wird der Ventilwiderstand gegen das Eintreten von Liquor. Je weniger die Feder drückt, desto geringer wird der Ventilwiderstand gegen das Eintreten von Liquor.

Der eindringende Liquor gelangt über eine Ablauföffnung und eine nicht dargestellte implantierte Schlauchleitung in den Bauchraum des Patienten.

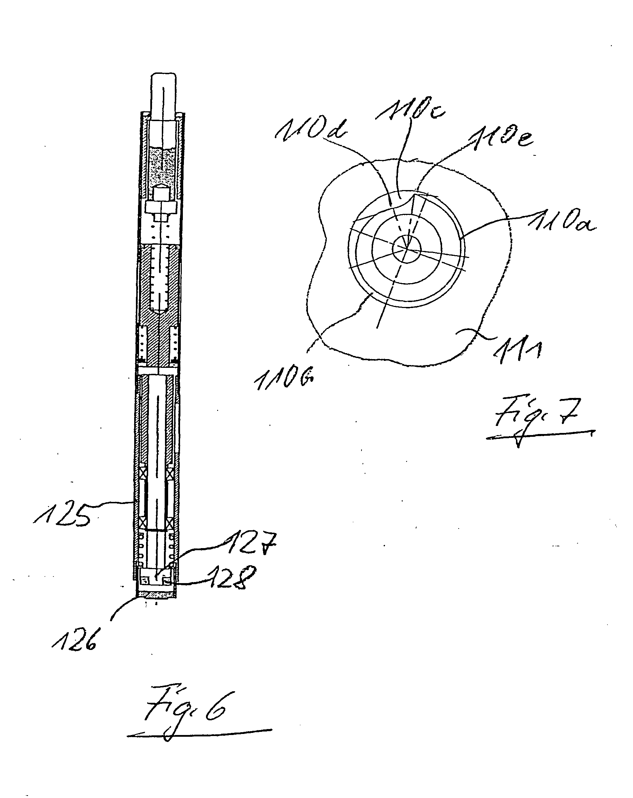

Der lange Hebelarm 107a erstreckt sich im Ausführungsbeispiel bis zur Mitte des zylindrischen Ringes 101 und gleitet dort auf einer Kurvenbahn 110. Die Kurvenbahn befindet sich in einem Schlitz. Sie bildet dort den Boden des Schlitzes und ist Bestandteil eines zylindrischen Formteiles 111 als Verstellteller, nachfolgend als Formteil bezeichnet. Die eine seitliche Begrenzung des Schlitzes wird durch das zylindrische Formteil 111 gebildet. Die andere seitliche Begrenzung des Schlitzes wird durch eine Scheibe 112 gebildet. Fig. 6 zeigt Einzelheiten. Dort ist ein Ausschnitt des Formteiles 111 mit der Kurvenbahn 110a und der seitlichen Begrenzung 110b dargestellt, welche durch das Formteil 111 selbst gebildet wird.

Die Kurvenbahn 110a hat einen Anfang 110d und ein Ende 110e. Durch Drehung des Formteiles 111 im Uhrzeigersinn wird der Hebelarm 107a in der Ebene der Fig. 6 nach links gedrückt. Dadurch erhöht sich die Federspannung und entsteht ein größerer Druck auf die Ventilkugel 105.

Bei einer Bewegung des Formteiles 111 entgegen dem Uhrzeigersinn reduziert sich die Federspannung und verringert sich der Druck auf die Ventilkugel 105.

Die Scheibe 112 sitzt auf einem Absatz des Formteiles 112 und ist dort in nicht dargestellter Form befestigt.

Im übrigen ist zwischen dem Ende 110e und dem Anfang 11 0d der Kurvenbahn eine Verbindung 110c vorgesehen, so daß das Formteil bei Erreichen des Endes 110e einfach weiter gedreht werden kann, um wieder zu dem Anfang 110d zu gelangen.

Das Formteil 111 ist drehbeweglich auf einem Lagerbolzen 112 angeordnet und dort mit einer Ring 114 gesichert.

Das Formteil 111 besitzt im Ausführungsbeispiel Bohrungen 115 und 116 für einander diametral gegenüberliegende Permanentmagneten. Die Permanentmagneten sind als Stiftmagneten ausgebildet. Ihr Durchmesser liegt im Ausführungsbeispiel bei 2 mm. Die Magnete werden durch Deckel 117 in den Ausnehmungen 115 und 116 gehalten. Ihr Abstand von dem Stahlboden 102 des Ventilgehäuses ist gering.

Zur Einstellung des Ventildruckes wird das Formteil 111 gedreht und je nach Bedarf der Federdruck erhöht oder verringert. Die Einstellung erfolgt durch Drehung des Formteiles 11.

Zur Drehung des Formteiles dient eine Verstelleinrichtung wie sie in Fig. 7 dargestellt ist.
Zu der Verstelleinrichtung gehört ein Gehäuse 125 mit einer Kappe 126, mit der die Verstelleinrichtung auf dem Ventil aufgesetzt wird.

In dem Gehäuse 125 ist ein Kopf 127 mit zwei Stiftmagneten 128 vorgesehen. Die Stiftmagneten 128 besitzen den gleichen Abstand wie die Magneten des Formteiles 111, sind aber so angeordnet, daß sie beim Aufsetzen der Verstelleinrichtung auf dem Ventil mit anderen Polen zu den Magneten des Formteiles 111 weisen. Dadurch ziehen sich die Magnete an und folgt das Formteil 111 einer Drehung bzw. einer Schwenkbewegung der Verstelleinreichtung durch eine gleichsinnige Drehung bzw. gleichsinnige Schwenkung. Vorteilhafterweise erleichtert sich auch die genaue Positionierung der Verstelleinrichtung. Bei leichter Berührung führt die Anziehungskraft der Magneten die Verstelleinrichtung in die richtige Position.

Anschließend wird die Andrückung verstärkt, um eine geringfügige Verformung des Ventilgehäuses zu verursachen. Dabei wird der Gehäuseboden oder Gehäusedeckel elastisch verfomt. Um die Verformung zu erleichtert ist der Boden 2 mit einer Verformungsdicke versehen. Die Verformungsdicke beträgt im Ausführungsbeispiel 0,2 mm. Die Folge der Verformung ist ein Abheben des Formteiles 111 von den zugehörigen Reibungsflächen. Die Reibung wird aufgehoben. Entsprechend leicht läßt sich das Formteil drehen oder schwenken.
Um der erfindungsgemäßen Verformung Rechnung zu tragen ist in dem Gehäuse ein entsprechender Freiraum geschaffen. Dazu ist in dem Deckel 103 eine Vertiefung für den Ring 114 und den Bolzen 113 vorgesehen.

Zum Andrücken ist in der Verstelleinrichtung eine Mechanik vorgesehen, ähnlich einem Kugelschreiber. Die Mechanik bewirkt eine federnde Andrückung. Die Feder sichert das Ventilgehäuse vor übermäßiger Verformung.
Dabei können die Magnete mit der Kugelschreibermechanik in der zum Verstellen erforderlichen Position in der Kappe 126 arretiert werden bzw. nach erfolgter Verstellung wieder zurückgezogen werden. Das verhindert beim Abnehmen der Verstelleinrichtung, daß durch ungeschickte Bewegung der Verstelleinrichtung bereits eine unerwünschte weitere Verstellung stattfindet.

Im Ausführungsbeispiel ist die Andrückung des Kopfes 127 in der Kappe 126 durch entsprechende Auslegung des Federsystems so gewählt, daß eine Drehung des Gehäuses 125 durch den behandelnden Arzt zur Mitnahme des Kopfes 127 führt.
In anderen Ausführungsbeispielen ist zusätzlich oder alternativ eine Führung des Kopfes 127 vorgesehen, welche allein oder zusammen mit einer Andrückung die beschriebene Mitnahme des Kopfes 127 bei Drehung des Gehäuses 126 verursacht.
Für die Führung ist in einem Ausführungsbeispiel eine Nut- und Feder-Verbindung vorgesehen, welche die axiale Beweglichkeit ermöglicht, aber in Umfangsrichtung eine drehfeste Anordnung bewirkt.

## Patentansprüche

1. Einstellbares Hydrocephalusventil zum Druckausgleich des Liquor im Schädel eines Hydrocephaluspatienten,
wobei das Ventil dem Patienten implantiert werden kann und vorzugsweise über eine gleichfalls implantierte Schlauchleitung der überschüssige Liquor aus den Hirnkammern abgeleitet werden kann und
vorzugsweise in die obere Hohlvene oder in den Bauchraum drainiert werden kann,
wobei der Ventildruck durch eine Feder (4,10) bestimmt und die Feder (4,10) über eine Verstellmechanik verstellbar ist, die ein schwenkbewegliches oder drehbewegliches Teil (13) besitzt, das perkutan mittels Magneten verschwenkt oder gedreht werden kann, so daß die Feder (4,10) gespannt oder entlastet wird, und das arretiert werden kann,
**dadurch gekennzeichnet,**
**daß** als Arretierung eine in jeder Drehstellung durch Federdruck selbstaktivierende Bremse (8) vorgesehen ist,
wobei die Bremse durch Aufhebung des Federdruckes mechanisch und perkutan zur Ventilverstellung gelöst werden kann,
wobei ein federndes Ventilgehäuse verwendet wird, dessen Gehäusedeckel (18) die Feder für die Bremse (8) bildet,
wobei der Gehäusedeckel (18) in der Arretierungsstellung eine solche Form annimmt, daß die Verstelleinrichtung reibungsschlüssig an dem Gehäuse anliegt und daß der Gehäusedeckel (18) zur Verstellung so verformt wird, daß die Verstelleinrichtung ihren Reibungsschluß an dem Gehäuse verliert.

2. Ventil nach Anspruch 1,
**dadurch gekennzeichnet,**
**daß** die Gehäusewand (16,18,20,101,102,103) eine nach außen gewölbte Ausgangsform oder eine ebene Ausgangsform besitzt, welche durch die Verformung verringert wird oder eine ebene Form annimmt oder eine Einwärtswölbung annimmt
oder
die Gehäusewand (16,18,20,101,102,103) eine ebene Ausgangsform besitzt, die durch die Verformung eine Einwärtswölbung erfährt
oder
die Gehäusewand (16,18,20, 101,102, 103) eine einwärts gewölbte Ausgangsform besitzt und durch die Verformung eine weitere Einwärtswölbung erfährt.

3. Ventil nach Anspruch 1 oder 2,
**gekennzeichnet durch** die Verwendung eines drehbeweglichen oder schwenkbeweglich angeordneten Verstelltellers (1,111), der **durch** die Gehäuseverformung am Rand Reibungsschluß erfährt.

4. Ventil nach Anspruch 3,
**gekennzeichnet durch** einen mit der Gehäusewand (16,18,20,101,102,103) verspannbaren Verstellteller (1,111).

5. Ventil nach Anspruch 3 oder 4,
**dadurch gekennzeichnet,**
**daß** ein Verstellteller (1,111) verwendet wird, der im Querschnitt eine U-Form besitzt, so daß der Reibungsschluß an dem vorstehenden Rand der U-Form entsteht.

6. Ventil nach einem der Ansprüche 2 bis 5,
**gekennzeichnet durch** die Verwendung einer verformbaren Gehäusewand (16,18,20,101,102,103) mit einer Dicke bis 0,5mm.

7. Ventil nach Anspruch 6, **gekennzeichnet durch** eine verformbare Gehäusewand (16,18,20,101,102,103) mit einer Dicke bis 0,2mm.

8. Ventil nach einem der Ansprüche 2 bis 7, **gekennzeichnet durch** eine Verformung der Gehäusewand (16,18,20,101,102,103) für den Reibungsschluß bis zu einem Maß, das gleich dem zweifachen der Gehausewandstärke ist.

9. Ventil nach Anspruch 8, **gekennzeichnet durch** eine Verformung der Gehäusewand (16,18,20,101,102,103) bis 0,1mm.

10. Ventil nach einem der Ansprüche 1 bis 9,
**dadurch gekennzeichnet,**
**daß** das Gehäuse (16,18,20,101,102,103) aus Metall besteht.

11. Ventil nach Anspruch 10,
**dadurch gekennzeichnet,**
**daß** die verformbare Gehäusewand (16,18,20,101,102,103) aus Metall besteht.

12. Ventil nach Anspruch 10 oder 11,
**dadurch gekennzeichnet,**
**daß** das Metall Titan oder eine Titanlegierung ist.

13. Ventil nach einem der Ansprüche 1 bis 12,
**dadurch gekennzeichnet, daß** die Verformung des Gehäuses (16,18,20,101,102,103) zum Deaktivieren der Bremswirkung mittels einer außen liegenden Verstellvorrichtung erfolgt, deren Andruck weggesteuert und/oder druckgesteuert ist.

14. Ventil nach Anspruch 13, **gekennzeichnet durch** eine Druckmessung und eine Druckanzeige und/oder eine Druckbegrenzung
und eine Anpressung der Vorrichtung von Hand.

15. Ventil nach Anspruch 14, **gekennzeichnet durch** die Verwendung von Federgliedern für die Druckanzeige und die Druckbegrenzung und/oder die Verwendung von Dehnungsmeßstreifen für die Druckmessung.

16. Ventil nach einem der Ansprüche 13 bis 15, **gekennzeichnet durch** die Verwendung einer Druckvorrichtung, die zugleich als außen liegende Verstelleinrichtung ausgebildet ist.

17. Ventil nach einem der Ansprüche 1 bis 16,
**dadurch gekennzeichnet,**
**daß** die innen liegende Verstelleinrichtung und die außen liegende Verstelleinrichtung mit einem oder mehreren Magneten (2,3,35,36,50,128),
wobei die Magneten (50,128) der außen liegenden Verstelleinrichtung die die Magneten (2,3,35,36) der innen liegenden Verstelleinrichtung sich mit unterschiedlichen Polen gegenüberliegen, so daß ein Drehmoment von der außen liegenden Verstelleinrichtung auf die innen liegende Verstelleinrichtung übertragen wird.

18. Ventil nach Anspruch 17,
**dadurch gekennzeichnet,**
**daß** in der innen liegenden Verstelleinrichtung und der außen liegenden Verstelleinrichtung jeweils zwei Stift-Magnete (2,3,35,36,50,128) derart angebracht sind, daß von zwei korrespondierenden Magneten der eine Magnet den Südpol bildet und zur Bodenseite hin angeordnet ist und der andere Magnet den Nordpol bildet.

19. Ventil nach Anspruch 18, **gekennzeichnet durch** die Verwendung von Magneten (2,3,35,36,50,128) mit einem Durchmesser bis 3mm.

20. Ventil nach Anspruch 19, **gekennzeichnet durch** einen Durchmesser der Magneten bis 1 mm.

21. Ventil nach einem der Ansprüche 18 bis 20, **gekennzeichnet durch** eine Höhe der Magneten (2,3,35,36,50,128) bis 5mm.

22. Ventil nach Anspruch 21, **gekennzeichnet durch** eine Höhe der Magneten bis 2mm.

23. Ventil nach einem der Ansprüche 18 bis 22,
**dadurch gekennzeichnet,**
**daß** die Magnete (50,128) in der außen liegenden Verstelleinrichtung einen Abstand voneinander aufweisen, der von dem Abstand der Magnete (2,3,35,36) in der innen liegenden Verstelleinrichtung höchstens 3mm abweicht.

24. Ventil nach Anspruch 23,
**dadurch gekennzeichnet**, **dadurch gekennzeichnet,**
**daß** der Abstand höchstens 1mm abweicht.

25. Ventil nach Anspruch 23 oder 24,
**dadurch gekennzeichnet,**
**daß** die Magnete (2,3,35,36,50,128) höchstens einen Abstand von 20mm voneinander aufweisen.

26. Ventil nach Anspruch 25,
**dadurch gekennzeichnet,**
**daß** die Magnete (2,3,35,36,50,128) höchstens einen Abstand von 10mm voneinander aufweisen.

27. Ventil nach Anspruch 26, **gekennzeichnet durch** einen Abstand der Magnete von höchstens 8mm.

28. Ventil nach einem der Ansprüche 18 bis 27,
**dadurch gekennzeichnet,**
**daß** die Magnete (50,128) in der außen liegenden Verstelleinrichtung auf und ab bewegbar sind.

29. Ventil nach einem der Ansprüche 18 bis 28,
**dadurch gekennzeichnet,**
**daß** die außen liegende Verstelleinrichtung ein Gehäuse (101,102,103) und eine Verstellmechanik besitzt, die einzeln oder beide einem Kugelschreiber nachgebildet sind, wobei durch Andrücken und/oder durch drehfeste Anordnung der Magneten (50,128) im Gehäuse (50,128) der Verstelleinrichtung eine Drehung des Gehäuses (101,102,103) der Verstelleinrichtung auf die im Ventilgehäuse eingeschlossenen Magneten (2,3,35,36) übertragen wird.

30. Ventil nach einem der Ansprüche 18 bis 29,
**dadurch gekennzeichnet,**
**daß** die außen liegende Verstelleinrichtung perkutan mit einer Kappe
aufsetzbar ist.

31. Ventil nach einem der Ansprüche 18 bis 30,
**dadurch gekennzeichnet,**
**daß** die zur elastischen Verformung des Ventilgehäuses (16,18,20,101,102,103) vorgesehene Kraft über ein zwischengeschaltetes Federelement als Kraftbegrenzer mit der außen liegenden Verstelleinrichtung aufgebracht wird.

32. Ventil nach einem der Ansprüche 18 bis 31,
**dadurch gekennzeichnet,**
**daß** die außen liegende Verstelleinrichtung mit einer Messeinrichtung für die Verstellbewegung versehen ist.

33. Ventil nach Anspruch 32,
**dadurch gekennzeichnet,**
**daß** die Messeinrichtung eine Drehmeßeinrichtung ist.

34. Ventil nach einem der Ansprüche 18 bis 33,
**dadurch gekennzeichnet,**
**daß** die außen liegende Verstelleinrichtung sich frei auf die Magnetstellung im Ventil einstellt und daß die Drehstellung der Magneten (50,128) außen an der Verstelleinrichtung ablesbar ist.

35. Ventil nach Anspruch 34, **gekennzeichnet durch** ein Ablesefenster (53) im Gehäuse der außen liegenden Verstelleinrichtung.

36. Ventil nach einem der Ansprüche 1 bis 34,
**dadurch gekennzeichnet, daß** im Ventil ein Federstab als Feder (4) vorgesehen ist, der sowohl mit dem Verstellteller (1,111) als auch mit der Ventilkugel oder Ventilklappe des Ventils in Wirkverbindung steht, wobei die Schwenkebene des Federstabes parallel zu der Bewegungsebene des Verstelltellers (1,111) verläuft.

37. Ventil nach Anspruch 36,
**dadurch gekennzeichnet,**
**daß** der Federstab (4) durch einen metallischen Draht oder Blech erzeugt wird, dessen Querschnitt rund und/oder eckig ist und/oder blattförmig oder drahtförmig ist.

38. Ventil nach Anspruch 36 oder 37,
**dadurch gekennzeichnet,**
**daß** der Federstab (4) einen Durchmesser oder Dicke bis 0,5mm aufweist.

39. Ventil nach Anspruch 38,
gekenntzeichnet durch einen Durchmesser oder Dicke des Federstabs bis 0,3 mm.

40. Ventil nach Anspruch 39,
**gekennzeichnet durch** einen Durchmesser oder Dicke des Federstabs bis 0,2 mm.

41. Ventil nach einem der Ansprüche 36 bis 40, daß der Federstab (4) einen doppelarmigen Hebelarm bildet, dessen einer Hebelarm mit dem Verstellteller (1,111) und dessen anderer Hebelarm mit der Ventilkugel (5) oder Ventilklappe des Ventils in Wirkverbindung steht.

42. Ventil nach Anspruch 41,
**dadurch gekennzeichnet,**
**daß** der Federstab (4) gelenkig gelagert ist und sowohl gleitend gegen eine Kurvenscheibe des Verstelltellers (1,111) drückt und darüber hinaus gleitend gegen die Ventilkugel (5) oder Ventilklappe des Ventils drückt.

43. Ventil nach einem der Ansprüche 36 bis 42,
**dadurch gekennzeichnet,**
**daß** die Hebelarme des einen doppelarmigen Hebelarm bildenden Federstabes (4) zwischen sich einen Winkel einschließen, der kleiner 180 Grad ist.

44. Ventil nach Anspruch 43,
**gekennzeichnet durch** einen Winkel zwischen den Hebelarmen kleiner 90 Grad.

45. Ventil nach einem der Ansprüche 42 bis 44,
**dadurch gekennzeichnet,**
**daß** die Kurvenscheibe (13) an dem Verstellteller (1,111) zumindest teilweise spiralförmig verläuft, so daß bei Verdrehung des Verstelltellers (1,111) die Federvorspannung gleitend verändert wird.

46. Ventil nach Anspruch 45,
**dadurch gekennzeichnet,**
**daß** die Federvorspannung gleichmäßig oder ungleichmäßig verändert wird.

47. Ventil nach einem der Ansprüche 42 bis 46,
**dadurch gekennzeichnet,**
**daß** die Kurvenbahn an der Kurvenscheibe (13) sich über weniger als 360 Grad erstreckt und die Enden der Kurvenbahn durch Anschläge begrenzt sind, so daß sich durch die Verstellung des Verstelltellers (1,111) eine hin-und hergehende Schwenkbewegung ergeben kann
oder
**daß** die Kurvenbahn sich über einen Umfangswinkel von mindestens 300 Grad erstreckt und zwischen beiden Enden des spiralförmigen Teiles ein Übergang vorgesehen ist, so daß die Feder nach Erreichen einer Extremstellung durch fortschreitendes gleichsinniges Drehen des Verstelltellers (1,111) in die andere Extremstellung kommt.

48. Ventil nach Anspruch 47,
**dadurch gekennzeichnet,**
**daß** die Kurvenbahn (13) einen Verstellhub von 0,1 bis 2mm besitzt.

49. Ventil nach Anspruch 48, **gekennzeichnet durch** einen Verstellhub von 0,5 bis 0,9mm

50. Ventil nach einem der Ansprüche 42 bis 49,
**dadurch gekennzeichnet, daß** die Kurvenbahn (13) dem Verstellweg und/oder dem Verstelldruck angepasst ist.

51. Ventil nach einem der Ansprüche 1 bis 50,
**dadurch gekennzeichnet,**
**daß** die Feder (4) schwenkbeweglich gelagert ist.

52. Ventil nach Anspruch 51,
**dadurch gekennzeichnet,**
**daß** die Feder mit einem Stift versehen ist, der an jedem Ende in eine Ausnehmung des Gehäuses (2,3,35,36,101,102,103) und/oder von dessen Boden und/oder Deckel greift.

53. Ventil nach Anspruch 52,
**dadurch gekennzeichnet,**
**daß** der Stift mit Spitzen versehen ist und auf den Spitzen in den Ausnehmungen schwenkt.

54. Ventil nach Anspruch 52 oder 53,
**dadurch gekennzeichnet, daß** der Stift mit der Feder verschweißt oder verlötet ist.

55. Ventil nach einem der Ansprüche 51 bis 54,
**dadurch gekennzeichnet,**
**daß** der Stift einen Durchmesser bis 3mm aufweist.

56. Ventil nach Anspruch 55, **gekennzeichnet durch** einen Durchmesser des Stifts bis 2mm

57. Ventil nach Anspruch 56, **gekennzeichnet durch** einen Durchmesser des Stifts bis 1mm.

58. Ventil nach einem der Ansprüche 1 bis 57, **gekennzeichnet durch** eine flächige Berührung zwischen der Feder (4) und der Ventilkugel (5) oder der Ventilklappe.

59. Ventil nach Anspruch 52,
**dadurch gekennzeichnet,**
**daß** der Querschnitt der Feder (4) im Berührungsbereich mit der Ventilkugel (5) oder Ventilklappe als Blech ausgebildet ist.

60. Ventil nach einem der Ansprüche 42 bis 59,
**dadurch gekennzeichnet,**
**daß** bei einer Ausbildung der Feder (4) als doppelarmiger Hebelarm die einzelnen Arme unterschiedlichen Querschnitt besitzen.

61. Ventil nach einem der Ansprüche 1 bis 60,
**dadurch gekennzeichnet,**
**daß** Schweißverbindungen an dem Ventil Laserschweißungen sind.

62. Ventil nach einem der Ansprüche 1 bis 61,
**dadurch gekennzeichnet,**
**daß** das Gehäuse (16,18,20,101,102,103) zumindest einen lösbaren Deckel oder Boden besitzt und am Deckel oder Boden ein Lagerbolzen (7) für den Verstellteller (1,111) vorgesehen ist, wobei zu dem Gehäuse ein Gehäusering gehört, in dem eine Zulauföffnung (11) und eine Ablauföffnung (12) vorgesehen sind, wobei mindestens in einer Öffnung eine Ventilkugel (5) sitzt, die von der Feder in Funktionsstellung gehalten wird.

63. Ventil nach Anspruch 62,
**dadurch gekennzeichnet,**
**daß** der den Lagerbolzen (7) tragende Deckel oder Boden zur Deaktivierung der Bremse nach innen gedrückt wird.

64. Ventil nach Anspruch 62 und 63,
**dadurch gekennzeichnet,**
**daß** der Lagerbolzen (7) beim Einwärtsdrücken des ihn tragenden Deckels oder Bodens den Verstellteller von den Reibungsflächen im Gehäuse abhebt,
wobei der Verstellteller (1,111) in dem Gehäuse (16,18,20,101,102,103) ein Bewegungsspiel hat.

65. Ventil nach einem der Ansprüche 1 bis 64,
**dadurch gekennzeichnet,**
**daß** das Ventil einen Außendurchmesser bis 31mm und/oder eine Höhe bis 10mm besitzt.

66. Ventil nach Anspruch 65, **gekennzeichnet durch** einen Außendurchmesser bis 20mm und/oder eine Höhe bis 6mm.

## Claims

1. Adjustable hydrocephalus valve to equalise the pressure of the fluid in the cranium of a hydrocephalus patient,
wherein the valve can be implanted in the patient and the excess fluid can be diverted from the ventricles of cerebrum in the cranium of the patient preferably by means of a likewise implantable flexible tube and
preferably drained into the upper vena cava or into the abdominal cavity of the patient,
wherein the valve pressure is determined by a spring (4,10) and the spring (4,10) can be adjusted by an adjusting mechanism that possesses a pivotably movable or rotatably movable element (13) that is moved from the exterior by pivoting or turning by means of magnets, such that the spring (4,10,) is wound up or released, and can be locked,
**characterised in that**
a brake (8) that is self-activating by spring pressure in each rotational position is provided as the lock
wherein the brake can be mechanically and percutaneously disengaged by removing the spring pressure so as to adjust the valve
wherein a spring-loaded valve housing is used, whose housing cover (18) forms the spring for the brake (8),
wherein the housing cover in the locked position assumes a shape such that the adjustment device lies friction locked on the housing and that for adjustment, the housing cover (18) is deformed such that the adjustment device loses its frictional lock on the housing.

2. Valve according to Claim 1,
**characterised in that**
the wall of the housing (16,18,20,101,102,103) has a convex starting shape or a flat starting shape which is diminished by the deformation or assumes a flat shape or assumes a concave shape
or
the wall of the housing (16,18,20,101,102,103) has a flat starting shape, which experiences a concave shape when deformed
or
the wall of the housing (16,18,20,101,102,103) has a concave starting shape and experiences a more pronounced concave shape when deformed.

3. Valve according to Claim 1 or 2,
**characterised by** the use of a rotatably or pivotably movable adjustment plate (1,111) that experiences friction locking on the periphery due to the deformation of the housing.

4. Valve according to Claim 3,
**characterised by** an adjustment plate (1,111) that can be distorted with the wall of the housing (16,18,20,101,102,103).

5. Valve according to Claims 3 or 4,
**characterised in that**
an adjustment plate (1,111) is used that possesses a U shaped cross section, such that the friction locking results on the projecting edge of the U shape.

6. Valve according to one of Claims 2 to 5,
**characterised by** the use of a deformable housing wall (16,18,20,101,102,103) with a thickness of up to 0.5 mm.

7. Valve according to Claim 6, **characterised by** a deformable housing wall (16,18,20,101,102,103) with a thickness of up to 0.2 mm.

8. Valve according to one of Claims 2 to 7, **characterised by** a deformation of the housing wall (16,18,20,101,102,103) for the frictional lock up to a degree that is equal to twice the wall thickness of the housing.

9. Valve according to Claim 8, **characterised by** a deformation of the housing wall (16,18,20,101,102,103) of up to 0.1 mm.

10. Valve according to one of Claims 1 to 9,
**characterised in that,**
the housing (16,18,20,101,102,103) consists of metal.

11. Valve according to Claim 10,
**characterised in that,**
the deformable housing wall (16,18,20,101,102,103) consists of metal.

12. Valve according to Claim 10 or 11,
**characterised in that**
the metal is titanium or a titanium alloy.

13. Valve according to one of Claims 1 to 12,
**characterised in that** the deformation of the housing (16,18,20,101,102,103) for deactivating the effect of the brake is pressure controlled by means of an external adjustment device, whose contact pressure is controlled and/or pressure controlled.

14. Valve according to Claim 13, **characterised by** a pressure measurement and
a pressure gauge and/or a pressure limit
and a contact pressure of the device by hand.

15. Valve according to Claim 14, **characterised by** the use of spring members to indicate the pressure and the pressure limitation and/or the use of elongating measurement strips for measuring the pressure.

16. Valve according to one of Claims 13 to 15, **characterised by** the use of a pressure device that at the same time is designed as the external adjustment device.

17. Valve according to one of Claims 1 to 16,
**characterised in that**
the internal adjustment device and the external adjustment device are equipped with one or more magnets (2,3,35,36,50,128), wherein the magnets (50,128) of the external adjustment device are facing the magnets (2,3,35,36) of the internal adjustment device with different poles, such that a torque is transferred from the external adjustment device onto the internal adjustment device.

18. Valve according to Claim 17, **characterised in that** two pin magnets (2,3,35,36,50,128), are fitted into each of the internal adjustment device and the external adjustment device in such a way that of two corresponding magnets, the one magnet forms the south pole and is directed towards the bottom side and the other magnet forms the north pole.

19. Valve according to Claim 18, **characterised by** the use of magnets (2,3,35,36,50,128) with a diameter of up to 3 mm.

20. Valve according to Claim 19, **characterised by** a diameter of the magnets of up to 1 mm.

21. Valve according to one of Claims 18 to 20, **characterised by** a height of the magnets (2,3,35,36,50,128) of up to 5 mm.

22. Valve according to Claim 21, **characterised by** a height of the magnets of up to 2 mm.

23. Valve according to one of Claims 18 to 22,
**characterised in that**
the magnets (50,128) in the external adjustment device have a separation distance from one another, which differs from the separation distance of the magnets (2,3,35,36) in the internal adjustment device by 3 mm at the most.

24. Valve according to Claim 23,
**characterised in that**
the separation distance differs by 1 mm at the most.

25. Valve according to Claim 23 or 24,
**characterised in that**
the magnets (2,3,35,36,50,128) have a separation distance from one another of 20 mm at the most.

26. Valve according to Claim 25,
**characterised in that**
the magnets (2,3,35,36,50,128) have a separation distance from one another of 10 mm at the most.

27. Valve according to Claim 26, **characterised by** a separation of the magnets of 8 mm at the most.

28. Valve according to one of Claims 18 to 27,
**characterised in that**
the magnets (50,128) in the external adjustment device are movable to and fro.

29. Valve according to one of Claims 18 to 28,
**characterised in that**
the external adjustment device possesses a housing (101,102,103) and an adjustment mechanism, which individually or both are designed like a ball pen, wherein by pressing and/or by torque proof assembly of the magnets (50,128) in the housing (50,128) of the adjustment device, a rotation of the housing (101,102,103) of the adjustment device is transmitted to the magnets (2,3,35,36) enclosed in the valve housing.

30. Valve according to one of Claims 18 to 29,
**characterised in that**
the external adjustment device can be percutaneously attached with a cap.

31. Valve according to one of Claims 18 to 30,
**characterised in that**
the force envisaged for the elastic deformation of the valve housing (16,18,20,101,102,103) is applied through an interposed spring element as the force limiter with the external adjustment device.

32. Valve according to one of Claims 18 to 31,
**characterised in that**
the external adjustment device is provided with a measuring device for the adjustment movement.

33. Valve according to Claim 32,
**characterised in that**
the measuring device is a rotational measuring device.

34. Valve according to one of Claims 18 to 33,
**characterised in that**
the external adjustment device adjusts freely to the magnet position in the valve and that the rotational position of the magnets (50, 128, 315) can be read externally on the adjustment device.

35. Valve according to Claim 34, **characterised by** a reading window (53) in the housing of the external adjustment device.

36. Valve according to one of Claims 1 to 34,
**characterised in that** a spring member is provided as the spring (4) in the valve, said spring being operationally connected both with the adjustment plate (1,111) and also with the valve ball or valve cover of the valve, wherein the rotatable plane of the spring bar runs parallel to the plane of movement of the adjustment plate (1,111).

37. Valve according to Claim 36,
**characterised in that**
the spring bar (4) is produced by a metallic wire or sheet, whose cross section is circular and/or angular and/or blade shaped or wire shaped.

38. Valve according to Claim 36 or 37,
**characterised in that**
the spring bar (4) has a diameter or thickness of up to 0.5 mm.

39. Valve according to Claim 38,
**characterised by** a diameter or thickness of the spring bar (4) of up to 0.3 mm.

40. Valve according to Claim 39,
**characterised by** a diameter or thickness of the spring bar of up to 0.2 mm.

41. Valve according to one of Claims 36 to 40, in that the spring bar (4) forms a double armed lever arm, of which one lever arm operatively connects with the adjustment plate (1,111) and whose other lever arm operatively connects with the valve ball (5) or valve cap of the valve.

42. Valve according to claim 41,
**characterised in that**
the spring bar (4) is flexibly mounted and presses both in a sliding contact against a cam disk of the adjustment plate (1,111) and moreover presses in a sliding contact against the valve ball (5) or valve cap of the valve.

43. Valve according to one of Claims 36 to 42,
**characterised in that**
the lever arms of the spring bar (4) formed by a double-armed lever arm enclose an angle that is less than 180 degrees.

44. Valve according to Claim 43,
**characterised by** an angle between the lever arms of less than 90 degrees.

45. Valve according to one of Claims 42 to 44,
**characterised in that**
the cam disk (13) on the adjustment plate (1,111) runs at least partially spirally, such that on rotating the adjustment plate (1,111) the spring pre-tensioner is slidingly changed.

46. Valve according to Claim 45,
**characterised in that**
the spring pre-tensioner is uniformly or non-uniformly changed.

47. Valve according to one of Claims 42 to 46,
**characterised in that**
the cam track on the cam disk (13) extends over less than 360 degrees and the ends of the cam track are limited by arresters, such that a back and forth pivoting movement can result when adjusting the adjustment plate (1,111)
or
the cam track extends over a peripheral angle of at least 300 degrees and a transition is provided between both ends of the spiral part, such that the spring, on attaining an extreme position, comes into the other extreme position by gradual rotation of the adjustment plate (1,111) in the same direction.

48. Valve according to Claim 47,
**characterised in that**
the cam track (13) possesses an adjustment travel of 0.1 to 2 mm.

49. Valve according to Claim 48, **characterised by** an adjustment travel of 0.5 to 0.9 mm.

50. Valve according to one of claims 42 to 49,
**characterised in that** the cam track (13) is aligned to the adjustment path and/or to the adjustment pressure.

51. Valve according to one of Claims 1 to 50,
**characterised in that,**
the spring (4) is pivotably mounted.

52. Valve according to Claim 51,
**characterised in that**
the spring is provided with a bar, that at each end reaches into a recess of the housing (2,3,35,36,101,102,103) and/or reaches for its floor and/or cover.

53. Valve according to Claim 52,
**characterised in that**
the bar is provided with tips and pivots on the tips in the recesses.

54. Valve according to Claim 52 or 53,
**characterised in that** the bar is welded or soldered to the spring.

55. Valve according to one of Claims 51 to 54,
**characterised in that,**
the diameter of the bar is up to 3 mm.

56. Valve according to Claim 55, **characterised by** a diameter of the bar of up to 2 mm.

57. Valve according to Claim 56, **characterised by** a diameter of the bar of up to 1 mm.

58. Valve according to one of Claims 1 to 57, **characterised by** a flat contact between the spring (4) and the valve ball (5) or the valve cover.

59. Valve according to Claim 52,
**characterised in that**
the cross section of the spring (4) in the contact area with the valve ball (5) or valve cover is designed as a sheet.

60. Valve according to one of Claims 42 to 59,
**characterised in that**
for a design of the spring (4) as a double-arm lever arm, the individual arms have different cross sections.

61. Valve according to one of Claims 1. to 60,
**characterised in that**
welded joints on the valve are laser welds.

62. Valve according to one of Claims 1 to 60,
**characterised in that**
the housing (16,18,20,101,102,103) possesses at least one removable cover or floor and a bearing bolt (7) for the adjustment plate (1,111) is provided on the cover or floor, wherein a housing ring belongs to the housing, in which
an inlet opening (11) and an outlet opening (12) are provided, wherein a valve ball (5) sits in at least one opening, said ball being held in functional position by the spring.

63. Valve according to Claim 62,
**characterised in that**
the cover or floor carrying the bearing bolt (7) is pressed inwards for deactivating the brake.

64. Valve according to Claim 62 and 63,
**characterised in that**
on pressing in the cover or floor carrying the bearing bolt (7), said bolt lifts off the adjustment plate from the friction surfaces in the housing, wherein the adjustment plate (1,111) has a movement play in the housing (16,18, 20,101,102,103).

65. Valve according to one of Claims 1 to 64,
**characterised in that,**
the valve has an external diameter of up to 31 mm and/or a height of up to 10 mm.

66. Valve according to Claim 65, **characterised by** an external diameter of up to 20 mm and/or a height of up to 6 mm.

## Revendications

1. Valve d'hydrocéphalie réglable, pour l'équilibrage de pression de la liqueur dans le crâne d'un patient souffrant d'hydrocéphalie, la valve pouvant être implantée chez le patient et, de préférence, la liqueur en excès pouvant être évacuée hors des chambres cérébrales, par l'intermédiaire d'une conduite en tuyau souple également implantée, et de préférence être drainé dans la veine cave supérieure ou dans la cavité abdominale, la pression de valve étant déterminée au moyen d'un ressort (4, 10) et le ressort (4, 10) étant réglable par l'intermédiaire d'un mécanisme de réglage, comprenant une partie (13) mobile en pivotement ou mobile en rotation, pouvant être pivotée ou tournée de façon percutanée, au moyen d'aimants, de manière que le ressort (4, 10) puisse être tendu ou déchargé, et qui puisse être arrêté, **caractérisée en ce qu'**est prévu, comme blocage, un frein (8) s'auto-activant en toute position de rotation, par une pression élastique, le frein pouvant être relâché par suppression de la pression élastique, de façon mécanique et percutanée par rapport au réglage de valve, un boîtier de valve élastique étant utilisé, dont le couvercle de boîtier (18) forme le ressort pour le frein (8), le couvercle de boîtier (18) prenant, en position de blocage, une forme telle que le dispositif de réglage appuie, par une liaison avec friction, sur le boîtier, et **en ce que** le couvercle de boîtier (18) est, pour le réglage, déformé de manière que le dispositif de réglage perde sa liaison par friction sur le boîtier.

2. Valve selon la revendication 1, **caractérisée en ce que** la paroi de boîtier (16, 18, 20, 101, 102, 103) présente une forme initiale incurvée vers l'extérieur ou une forme initiale plane, diminuée par la déformation, ou prend une forme plane ou une incurvation vers l'intérieur, ou la paroi de boîtier (16, 18, 20, 101, 102, 103) présente une forme initiale plane, subissant une incurvation vers l'intérieur du fait de la déformation, ou la paroi de boîtier (16, 18, 20, 101, 102, 103) présente une forme initiale incurvée vers l'intérieur et subit une incurvation vers l'intérieur supplémentaire du fait de la déformation.

3. Valve selon la revendication 1 ou 2, **caractérisée par** l'utilisation d'un disque de valve (1, 111) disposé de façon mobile en rotation ou mobile en pivotement, subissant une liaison par friction sur le bord, du fait de la déformation du boîtier.

4. Valve selon la revendication 3, **caractérisée par** un disque de valve (1, 111), susceptible d'être serré à la paroi de boîtier (16, 18, 20, 101, 102, 103).

5. Valve selon la revendication 3 ou 4, **caractérisée en ce qu'**on utilise un disque de valve (1, 111) ayant, en vue en coupe transversale, une forme en U de manière que la liaison par friction se crée sur le bord en saillie de la forme en U.

6. Valve selon l'une des revendications 2 à 5, **caractérisée par** l'utilisation d'une paroi de boîtier (16, 18, 20, 101, 102, 103) déformable, d'une épaisseur allant jusqu'à 0,5 mm.

7. Valve selon la revendication 6, **caractérisée par** l'utilisation d'une paroi de boîtier (16, 18, 20, 101, 102, 103) déformable, d'une épaisseur allant jusqu'à 0,2 mm.

8. Valve selon l'une des revendications 2 à 7, **caractérisée par** une déformation de la paroi de boîtier (16, 18, 20, 101, 102, 103), pour la liaison par friction, allant jusqu'à une valeur égale à deux fois l'épaisseur de la paroi de boîtier.

9. Valve selon la revendication 8, **caractérisée par** une déformation de la paroi de boîtier (16, 18, 20, 101, 102, 103) allant jusqu'à 0,1 mm.

10. Valve selon l'une des revendications 1 à 9, **caractérisée en ce que** le boîtier (16, 18, 20, 101, 102, 103) est composé de métal.

11. Valve selon la revendication 10, **caractérisée en ce que** la paroi de boîtier (16, 18, 20, 101, 102, 103) déformable est composée de métal.

12. Valve selon la revendication 10 ou 11, **caractérisée en ce que** le métal est du titane ou un alliage de titane.

13. Valve selon l'une des revendications 1 à 12,
**caractérisée en ce que** la déformation du boîtier (16, 18, 20, 101, 102, 103), pour la désactivation de l'effet de frein, est effectuée au moyen d'un dispositif de réglage situé extérieurement, dont la pression d'application est commandée par le biais d'une course de déplacement et/ou commandée par le biais d'une pression.

14. Valve selon la revendication 13, **caractérisée par** une mesure de pression et un affichage de pression et/ou une limitation de pression et un pressage du dispositif à la main.

15. Valve selon la revendication 14, **caractérisée par** l'utilisation d'organes élastiques, pour l'affichage de pression et la limitation de pression, et/ou l'utilisation de jauges extensométriques, pour la mesure de pression.

16. Valve selon l'une des revendications 13 à 15, **caractérisée par** l'utilisation d'un dispositif à pression, réalisé en même temps sous forme de dispositif de réglage placé extérieurement.

17. Valve selon l'une des revendications 1 à 16, **caractérisée en ce que** le dispositif de réglage placé intérieurement et le dispositif de réglage placé extérieurement comprennent un ou plusieurs aimants (2, 3, 35, 36, 50, 128), les aimants (50, 128) du dispositif de réglage placé extérieurement étant placés, avec des pôles différents, à l'opposé des aimants (2, 3, 35, 36) du dispositif de réglage placé intérieurement, de sorte qu'un couple de rotation est transmis du dispositif de réglage placé extérieurement au dispositif de réglage placé intérieurement.

18. Valve selon la revendication 17, **caractérisée en ce que,** dans le dispositif de réglage placé intérieurement et le dispositif de réglage placé extérieurement, sont respectivement montés deux aimants-tiges (2, 3, 35, 36, 50, 128), de manière que, de deux aimants correspondants, un aimant forme le pôle sud et soit disposé tourné vers le côté fond, et que l'autre aimant forme le pôle nord.

19. Valve selon la revendication 18, **caractérisée par** l'utilisation d'aimants (2, 3, 35, 36, 50, 128) d'un diamètre allant jusqu'à 3 mm.

20. Valve selon la revendication 19, **caractérisée par** un diamètre des aimants allant jusqu'à 1 mm.

21. Valve selon l'une des revendications 18 à 20, **caractérisée par** une hauteur des aimants (2, 3, 35, 36, 50, 128) allant jusqu'à 5 mm.

22. Valve selon la revendication 21, **caractérisée par** une hauteur des aimants allant jusqu'à 2 mm.

23. Valve selon l'une des revendications 18 à 22, **caractérisée en ce que** les aimants (50, 128) situés dans le dispositif de réglage placé extérieurement présentent un espacement mutuel différant, au plus de 3 mm, de l'espacement des aimants (2, 3, 35, 36) situés dans le dispositif de réglage placé intérieurement.

24. Valve selon la revendication 23, **caractérisée en ce que** l'espacement diffère au plus de 1 mm.

25. Valve selon la revendication 23 ou 24, **caractérisée en ce que** les aimants (2, 3, 35, 36, 50, 128) présentent au plus un espacement mutuel de 20 mm.

26. Valve selon la revendication 25, **caractérisée en ce que** les aimants (2, 3, 35, 36, 50, 128) présentent au plus un espacement mutuel de 10 mm.

27. Valve selon la revendication 26, **caractérisée par** un espacement maximal de 8 mm entre les aimants.

28. Valve selon l'une des revendications 18 à 27, **caractérisée en ce que** les aimants (50, 128) situés dans le dispositif de réglage placé extérieurement sont déplaçables en levée et en descente.

29. Valve selon l'une des revendications 18 à 28, **caractérisée en ce que** le dispositif de réglage placé extérieurement comprend un boîtier (101, 102, 103) et un mécanisme de réglage, reproduisant, individuellement ou les deux, un stylo à bille, où, par pressage et/ou par un agencement, assujetti en rotation, des aimants (50, 128) dans le boîtier (50, 128) du dispositif de réglage, une rotation du boîtier (101, 102, 103) du dispositif de réglage est transmise aux aimants (2, 3, 35, 36) enfermés dans le boîtier de valve.

30. Valve selon l'une des revendications 18 à 29, **caractérisée en ce que** le dispositif de réglage placé extérieurement est applicable, de manière percutanée, avec un capuchon.

31. Valve selon l'une des revendications 18 à 30, **caractérisée en ce que** la force, prévue pour la déformation élastique du boîtier de valve (16, 18, 20, 101, 102, 103), est appliquée avec le dispositif de réglage placé extérieurement, par l'intermédiaire d'un élément élastique interposé faisant office de limiteur de force.

32. Valve selon l'une des revendications 18 à 31, **caractérisée en ce que** le dispositif de réglage placé extérieurement est muni d'un dispositif de mesure, pour la mesure de la course de réglage.

33. Valve selon la revendication 32, **caractérisée en ce que** le dispositif de mesure est un dispositif de mesure de rotation.

34. Valve selon l'une des revendications 18 à 33, **caractérisée en ce que** le dispositif de réglage placé extérieurement se règle librement à la position d'aimant dans la valve, et **en ce que** la position en rotation des aimants (50, 128) est lisible extérieurement sur le dispositif de réglage.

35. Valve selon la revendication 34, **caractérisée par** une fenêtre de lecture (53), placée dans le boîtier du dispositif de réglage placé extérieurement.

36. Valve selon l'une des revendications 1 à 34, **caractérisée en ce que**, dans la valve, est prévue une barre élastique se présentant sous forme de ressort (4), relié(e) fonctionnellement tant au disque de valve (1, 111) qu'également à la bille de valve ou au clapet de la valve, le plan de pivotement de la barre élastique s'étendant parallèlement au plan de déplacement du disque de valve (1, 111).

37. Valve selon la revendication 36, **caractérisée en ce que** la barre élastique est produite par un fil métallique ou une tôle, dont la section transversale est ronde et/ou angulaire et/ou en forme de lame ou en forme de fil.

38. Valve selon la revendication 38 ou 37, **caractérisée en ce que** la barre élastique (4) présente un diamètre ou épaisseur allant jusqu'à 0,5 mm.

39. Valve selon la revendication 38, **caractérisée en ce que** la barre élastique présente un diamètre ou épaisseur allant jusqu'à 0,3 mm.

40. Valve selon la revendication 39, **caractérisée en ce que** la barre élastique présente un diamètre ou épaisseur allant jusqu'à 0,2 mm.

41. Valve selon l'une des revendications 36 à 40, **caractérisée en ce que** la barre élastique (4) forme un bras de levier à deux bras, dont un bras de levier est relié fonctionnellement au disque de valve (1, 111) et dont l'autre bras de levier est relié fonctionnellement à la bille de valve (5) ou au clapet de valve de la valve.

42. Valve selon la revendication 41, **caractérisée en ce que** la barre élastique (4) est montée de manière articulée et, presse avec glissement aussi bien contre un disque de came du disque de valve (1, 111) que, de plus, contre la bille de valve (5) ou un clapet de valve de la valve.

43. Valve selon l'une des revendications 36 à 42, **caractérisée en ce que** les bras de levier de la barre élastique (4) formant un bras de levier à double bras, ont entre eux un angle inférieur à 180 degrés.

44. Valve selon la revendication 43, **caractérisée par** un angle inférieur à 90 degrés entre les bras de levier.

45. Valve selon l'une des revendications 42 à 44, **caractérisée en ce que** le disque de came (13), sur le disque de valve (1, 111), s'étend au moins partiellement en forme de spirale, de manière que, lors d'une rotation du disque de valve (1, 111), la précontrainte élastique soit modifiée de manière glissante.

46. Valve selon la revendication 45, **caractérisée en ce que** la précontrainte élastique est modifiée de manière régulière ou irrégulière.

47. Valve selon l'une des revendications 42 à 46, **caractérisée en ce que** la piste de came, sur le disque de came (13), s'étend sur moins de 360 degrés et les extrémités de la piste de came sont limitées par des butées, de manière qu'un mouvement de pivotement en va et vient puisse être produit du fait du réglage du disque de valve (1, 111), ou **en ce que** le disque de came s'étend sur un angle périphérique d'au moins 300 degrés et une transition est prévue, entre les deux extrémités de la partie en forme de spirale, de manière que le ressort, après atteinte d'une position extrême, passe à l'autre position par une continuation de rotation, faite dans le même sens, du disque de valve (1, 111).

48. Valve selon la revendication 47, **caractérisée en ce que** la piste de came (13) présente une course de réglage dans la fourchette allant de 0,1 à 2 mm.

49. Valve selon la revendication 48, **caractérisée par** une course de réglage dans la fourchette allant de 0,5 à 0,9 mm.

50. Valve selon l'une des revendications 42 à 49, **caractérisée en ce que** la piste de came (13) est adaptée à la course de réglage et/ou à la pression de réglage.

51. Valve selon l'une des revendications 1 à 50, **caractérisée en ce que** le ressort (4) est monté avec une mobilité en pivotement.

52. Valve selon la revendication 51, **caractérisée en ce que** le ressort est muni d'une tige qui, à chaque extrémité, s'engage dans un évidement du boîtier (2, 3, 35, 36, 101, 102, 103) et/ou de son fond et/ou couvercle.

53. Valve selon la revendication 52, **caractérisée en ce que** la tige est munie de pointes et pivote sur les pointes, dans les évidements.

54. Valve selon la revendication 52 ou 53, **caractérisée en ce que** la tige est soudée ou brasée au ressort.

55. Valve selon l'une des revendications 51 à 54, **caractérisée en ce que** la tige présente un diamètre allant jusqu'à 3 mm.

56. Valve selon la revendication 55, **caractérisée en ce que** la tige présente un diamètre allant jusqu'à 2 mm.

57. Valve selon la revendication 56, **caractérisée en ce que** la tige présente un diamètre allant jusqu'à 1 mm.

58. Valve selon l'une des revendications 1 à 57, **caractérisée par** un contact à plat entre le ressort (4) et la bille de valve (5) ou le clapet de valve.

59. Valve selon la revendication 52, **caractérisée en ce que** la section transversale du ressort (4) est réalisée sous forme de tôle, dans la zone de contact avec la bille de valve (5) ou le clapet de valve.

60. Valve selon l'une des revendications 42 à 59, **caractérisée en ce que**, dans le cas de réalisation du ressort (4) sous forme de bras de levier à deux bras, les bras individuels présentent une section transversale différente.

61. Valve selon l'une des revendications 1 à 60, **caractérisée en ce que** des liaisons soudées, réalisées sur la valve, sont des soudures au laser.

62. Valve selon l'une des revendications 1 à 61, **caractérisée en ce que** le boîtier (16, 18, 20, 101, 102, 103) présente au moins un couvercle ou fond déslidarisable, et, sur le couvercle ou fond, est prévu un boulon (7) pour le disque de valve (1, 111), sachant qu'appartient au boîtier une bague de boîtier, dans laquelle sont prévues une ouverture d'alimentation (11) et une ouverture d'évacuation (12), au moins dans une ouverture étant placée une bille de valve (5) maintenue en position fonctionnelle par le ressort.

63. Valve selon la revendication 62, **caractérisée en ce que** le couvercle ou fond, portant le boulon de palier (7), est pressé vers l'intérieur, pour produire la désactivation du frein.

64. Valve selon les revendications 62 et 63, **caractérisée en ce que** lors du pressage, vers l'intérieur, du couvercle ou fond le portant, le boulon de palier (7) soulève le disque de réglage des faces de friction situées dans le boîtier, le disque de réglage (1, 111) ayant, dans le boîtier (16, 18, 20, 101, 102, 103), un jeu de mouvement.

65. Valve selon l'une des revendications 1 à 64, **caractérisée en ce que** la valve présente un diamètre extérieur allant jusqu'à 31 mm et/ou une hauteur allant jusqu'à 10 mm.

66. Valve selon la revendication 65, **caractérisée par** un diamètre extérieur allant jusqu'à 20 mm et/ou une hauteur allant jusqu'à 6 mm.
